# EUROPEAN PATENT APPLICATION

(11) **EP 1 972 692 A1**
(43) Date of publication of application: **24.09.2008**
(21) Application number: 07104386.3
(22) Date of filing: 19.03.2007
(51) Int. Cl.: C12Q 1/68, A01H 1/04, A01H 5/00

(54) **Methods of producing haploid and doubled haploid oil palms**

(71) Applicant: Sumatra Investment Corporation Pte. Ltd., 068911 Singapore (SG)
(72) Inventor: Nelson, Stephen, Peter, Conner, North Sumatra (ID); Dunwell, James, Martin, Reading, RG6 6AS (GB); Wilkinson, Michael, James, Aberystwyth, SY23 3DA (GB); Caligari, Peter, Douglas, Savaria, Reading RG6 5FY (GB)
(74) Representative: Gemmell, Peter Alan

(57) **Abstract**

The present invention relates to haploid oil palm plants and homozygous doubled haploid oil palm plants. The invention also relates to methods for producing and selecting haploid and doubled haploid plants. More particularly, but not exclusively, the method may be used for selecting haploid and doubled haploid oil palm plants. The seeds are first germinated and haploid plants are selected by a large-scale screening based on a combination of the phenotype of the seedlings, and the use of molecular methods combined with flow cytometry techniques to identify haploid and doubled haploid seedlings. More particularly, the method for selecting haploid and doubled haploid plants comprises: (a) germinating seeds; (b) selecting seedlings with desired phenotype; (c) isolating cells from the seedlings and determining the DNA content of the cells; and (d) isolating and purifying the DNA and using defined molecular markers to characterise the genotype of the plant. The haploid oil palm plants and homozygous doubled haploid oil palm plants may be used for various applications, such as producing homozygous doubled haploid oil palms, and the subsequent intercrossing of the homozygous doubled haploid oil palms to produce an F₁ hybrid generation.

## Description

### Field of the Invention

The present invention relates to haploid oil palm plants and homozygous doubled haploid oil palm plants. The invention also relates to methods for producing and selecting haploid and doubled haploid plants. More particularly, but not exclusively, the method may be used for selecting haploid and homozygous doubled haploid oil palm plants.

### Background of the Invention

Although plant breeding programs worldwide have made considerable progress developing new cultivars with improved yield, pest and disease resistance, and other useful traits, breeding as a whole relies on screening numerous plants to identify novel, desirable characteristics. Often, very large numbers of progeny from crosses must be grown and evaluated over several years in order to select one or a few plants with a desired combination of traits.

In a typical plant breeding programme, two parent plants are crossed and the resulting progeny are screened and one or more plants that possess a desirable combination of phenotypic traits are identified and selected. The plant with desired traits may then self-fertilise or be crossed to yield a population of progeny plants that must be individually analysed to determine which plants possess the desired combination of phenotypic traits originally introduced in the first generation. If, as is often the case, the desired phenotypic traits are derived from the combined effect of several genes, then the number of progeny plants that must be screened depends on the number of genetic differences between the parent plants. Thus, the greater the number of genetically-controlled differences between parents, the larger the number of progeny that must be grown and evaluated, and the lower the probability of obtaining progeny with all the desired traits. The problem is exacerbated when some of these traits (such as yield) require the plants to reach maturity before they can be evaluated.

One possible solution to the problem of screening large numbers of progeny that segregate for the desired traits depends on the ability to produce or identify haploid plants derived from the gametic cells of parental individuals. The chromosome complements of these haploids sometimes spontaneously double to produce diploid plants or else can be doubled artificially using colchicine or by other means. In particular, though not exclusively, doubled haploids can be produced by the *in vitro* culture of microspores that normally give rise to pollen grains. The resultant doubled haploid plants, however they are derived, are instantly and completely homozygous. This means that the seed offspring generated from the selfing of such plants are genetically identical to the parental clone and so can be multiplied rapidly by seed. Furthermore, when two such doubled haploids are crossed sexually, the resultant seed offspring are genetically invariant and heterozygous for all loci that differ between the two parents (i.e. they are genetically uniform F₁ offspring).

The ploidy level of a somatic cell is defined as the number of genome sets of chromosomes that it contains. A genome set of chromosomes (also known as the base number, x) is most simply described as the number of heterologous chromosomes present in the nuclear genome and equals that present in the gametophytes of a diploid organism. For example, humans are diploid organisms, having 2n=2x=46 chromosomes in their somatic cells and n=x=23 in their gametes (eggs and sperm). When the ploidy level is greater than one, genetic analysis is made more difficult by the effects of dominance; when more than one copy of a gene is present only one copy, the dominant one, may influence phenotype or else both copies contribute to the expressed phenotype (partial or no dominance). With dominance, the other copy of the gene, the recessive allele, is apparently 'masked' because its presence is not apparent at the phenotypic level. Haploid organisms contain the same number of chromosomes (n) in their somatic cells as do the normal gametes of the species. The term haploid sporophyte is generally used to designate sporophytes having the gametic chromosome number (Palmer and Keller, 2005a) and in a diploid organism this complement is the same as the base number (x).

Haploids of higher plants can be distinguished from their diploid equivalent in many ways. Most obviously from the perspective of phenotype, they are usually smaller in appearance, partly because of their smaller cell size; in general terms, cell volume in plants is positively correlated to ploidy level. Several methods for the provisional assignment of the haploid status of a plant exploit this relationship. The most widely used of these phenotypic methods is the measurement of stomatal guard cell length and chloroplast content in these cells (e.g. Sari et al., 1999; Stanys et al., 2006), although none of the phenotypic predictors of haploidy is absolutely reliable. Methods providing direct measurements of genome size provide a far more reliable diagnosis of haploid status. These include direct measurement of the chromosome number using conventional chromosome counting techniques, and measurement of the DNA content using microdensitometry (e.g. Zhang et al., 1999) or more especially, flow cytometry (Coba de la Pena and Brown, 2001; Bohanec, 2003; Eeckhaut et al., 2005). The latter technique has also been applied to characterise the cell cycle stages in various tissues of oil palm material, although not for the detection of haploid plants or tissues (Srisawat and Kanchanapoom 2005; Srisawat et al., 2005). It is also possible to exploit the absolute absence of heterozygosity in haploids and doubled haploids to detect such plants using various co-dominantly inherited molecular marker methods (e.g. Chani et al., 2000; Tang et al., 2006).

Haploids may have intrinsic value because of their overall reduction in size compared with diploids. Haploids also have value in allowing the isolation of mutants, which may be masked in a diploid, particularly where the mutant allele is non-functional. Haploids also have value in transformation programmes. If haploids are transformed directly, then true breeding diploid transgenic plants can be produced in one step following doubling of chromosomes. It should be noted that a wide range of techniques for chromosome doubling are known (Kasha, 2005 and references incorporated therein) and these techniques, or modifications of them, are applicable and relevant in the context of this invention. Some studies on the development of chromosome doubling techniques in oil palm have already been reported and whilst these data relate to the doubling of diploid material (to give polyploids), the protocol described will also have utility for haploid doubling (Madon et al., 2005a).

An important use of haploids is based on the fact that marked improvements in the economics of plant breeding can be achieved via doubled haploid production, since selection and other procedural efficiencies can be markedly improved through the provision of elite true-breeding (homozygous) progenies (Nei, 1963; Choo, 1981; Melchers, 1972; Hermsen and Ramanna, 1981; Snape, 1984). With doubled haploid production systems, homozygosity is achieved in one generation. Thus, the breeder can eliminate the numerous cycles of inbreeding that is usually necessary to achieve practical levels of homozygosity by conventional methods. Indeed, absolute homozygosity for all traits is not achievable by conventional breeding methods. Consequently, an efficient doubled haploid technology would enable breeders to reduce the time and the cost of cultivar development relative to conventional breeding practices.

Spontaneous haploids may occur in many species of plants, albeit at low frequencies. For tropical perennial crop species of commercial importance the following summary is relevant:- oil palm (none reported from any source), rubber (no spontaneous haploids reported, though two reports from anther and ovary culture quoted in Table 3-1 from Maluszynski et al., 2003b, are Chen et al., 1988, Jayasree et al., 1999), sugar cane (no spontaneous haploids, though again two reports quoted in Maluszynski et al., 2003b, are Liu et al., 1980, and Fitch and Moore 1996), coffee (reports of spontaneous haploids, eg Lashermes et al., 1994,) cotton (many examples of spontaneous haploids), cacao (spontaneous haploids reported, Dublin 1972). It is important to note in the context of the current invention that in none of the cases where spontaneous haploids have been described has it been subsequently possible to accumulate significant numbers of haploids or doubled haploids to have utility for crop improvement, in other words they are rare in occurrence. For this reason, emphasis has turned to alternative means of generating haploids and doubled haploids. Haploid plants of several other species have also been created following various laboratory manipulations, including parthenogenesis, androgenesis, chromosome elimination, and tissue culture-based methods, although progress has been poor for perennial crops, particularly tropical species that habitually outcross.

The general lack of progress towards haploid and doubled haploid production in woody species (Stettler and Howe, 1966) is due mainly to the present emphasis on production methods involving an *in vitro* phase; there are numerous problems associated with the general intransigence of woody species to growth under such conditions

As well as having value in their own right as potential new varieties, homozygous plants also have utility for the generation of F₁ hybrid plants, where crosses are made between selected homozygous males and females. These F₁ plants often exhibit so-called hybrid vigour (heterosis), a characteristic often associated with dramatic increases in yield compared with either parent, and first described by Shull (1908). Furthermore, the production of F₁ hybrids allows the breeder to produce large quantities of seed comprising of a single genotype from homozygous parental lines. This property will have many advantages over a genetically heterogeneous mix of genotypes because of the potential to select single elite genotypes that produce high yields and / or possess other desirable characteristics. There is also potential to achieve higher yields by selecting genotypes for adaptation to specific environments and to optimise agronomic and management practices. In many crops, the only realistic alternative to producing a single genotype in commercial quantities is by asexual cloning. There are well-developed methods of vegetative propagation, using suckers, cuttings or grafts to produce clones for some crops (for example, rubber, cocoa and coffee) but not all crops (for example, oil palm and coconuts).

According to Hermsen and Ramanna (1981):- "Just as in self-pollinators, the application of haploidy in cross-pollinated diploid crops is based on the use of DH-lines (Doubled Haploid lines). However, owing to inbreeding depression (note: homozygous individuals of a normally outcrossing species typically exhibit reduced vigour and this is known as inbreeding depression), these lines cannot be used directly but only as parental inbred lines for the production of hybrid varieties. When inbred lines are being developed via haploids, all barriers to repeated selfing, which are characteristics of natural cross-pollinators are bypassed, e.g. dioecy, self incompatibility and long juvenile periods. The time saving is particularly apparent in biennial crops and in crops with a long juvenile period. Only via haploidy can inbred lines be developed in these crops."

As such, haploid plants (and doubled haploid plants) reveal all their genetic information or, in other words, their genotype is completely displayed by their phenotype. Resistance to pest and diseases or unfavourable external factors (drought, salinity, heavy metal toxicity etc) can thus be directly recognized and selected. Haploid plants allow the detection of mutants that are unable to pass through the embryonic phases. For similar reasons haploid plant tissue make ideal vehicles for genetic transformation, by whatever gene manipulation techniques are relevant, to give genetically modified material that on doubling give homozygous versions of the introduced gene or genes.

The agricultural applications for haploids centres on their capacity for the rapid generation of homozygous genotypes after chromosome doubling:
■ Reduce time for variety development, e.g. 10 to 6 years or less;
■ Homozygous recombinant line can be developed in one generation instead of after numerous backcross generations; and
■ Selection for recessive traits in recombinant lines is more efficient because recessive alleles are not 'masked' by the effects of dominant alleles.
■ Introduction of "alien" genes speeded by allowing homozygotes to be developed readily.

The crossing of two homozygous elite lines (such as can be produced by doubling haploids) can generate genetically uniform, highly heterozygous 'hybrid' varieties, as is exemplified by the highly successful hybrid maize varieties produced in the USA during the 1930s. It is not surprising that there have been many efforts to reproduce the yield increase gained in hybrid maize varieties in other crops, through the development of 'hybrid lines'. For example, F₁ hybrid varieties of sunflower and sugar beet are now widely grown on a commercial basis, and hybrid lines of oilseed rape (canola) and rice are becoming increasingly available; more than half the rice grown in China is 'hybrid', with yields at least 20% higher than the non-hybrid equivalent. To date, there has been no corresponding progress with the highest yielding of all oilseed crops, oil palm; although oil yields of 4.8-7 t/ha are 3-8 times greater than other oil seed crops (Wahid et al., 2004). Overall, oil palm is the world's leading source of vegetable oils and fats, on a par with soybean (Abdullah, 2005) but has nevertheless yet to benefit from the release of hybrid varieties.

The lack of progress towards the generation of hybrid varieties for oil palm is principally because the breeding system of the crop precludes the simple production of inbred lines. Oil palm is essentially an outbreeding species, but unlike corn in which a male and a female flower are produced on the same plant at the same time, each oil palm plant produces either male or female flowers at any one time, and therefore a palm can only readily be self-pollinated by methods of controlled pollination using stored pollen. Progress in converting oil palm into a hybrid crop, and thereby exploiting the potential hybrid vigour, depends upon the development of a process for the reliable production of homozygous plants. To date, however, there is no published example of any haploid, or homozygous diploid oil palm plant. There has nevertheless been extensive breeding (Wahid et al., 2004), cell culture (Abdullah 2005; Abdullah et al., 2005; Rival and Parveez, 2005; Te-chato et al., 2005), and transformation studies (US Application 20030159175) geared towards the genetic improvement of the oil palm crop.

All oil palm seeds currently used for commercial plantings are produced from parents selected from genetically heterogeneous populations of non-homozygous palms. Variation in the level of parental heterozygosity and in the genetic divergence between parental lines means that there is extensive genetic segregation amongst the resulting seed offspring. Thus, the seeds produced from palm crosses are therefore not genetically uniform. This genetic variation impedes the oil palm industry from selecting specific genotypes for high yield or other desirable traits.

The oil palm only has a single growing point, and unlike some other palms species, does not produce suckers. For these reasons, clones cannot be produced by the standard methods of vegetative propagation. However, it is possible to produce somatic clones by tissue culture, in which small pieces of tissue (explants) from leaves, inflorescences or roots are first cultured on special nutrient media. The growing tissue may then form callus (a mass of cells without differentiation), and this may be treated to produce embryoids tissue, which themselves slowly develop into plant shoots. However, such tissue culture techniques are generally difficult and laborious to perform, and the underlying biology is poorly understood. Moreover, there is also a risk of somaclonal variation, induced by the tissue culture process itself and which has led to phenotypic abnormalities (Corley et al., 1986) that may result in complete loss of yield.

Mention should be made of the apparent claim by Maluszynski et al. (2003b, see Table 3-1) that Texeira et al. (1994) have already published a protocol for doubled haploid production in oil palm. This claim is erroneous. In fact, the latter publication describes somatic embryogenesis from diploid floral tissue, and does not describe the culture of anthers or other reproductive tissue to produce haploid embryos and plants.

The only related work on other palm species include failed attempts to produce haploids of coconut *(Cocos nucifera )* via anther culture (Thanh-Tuyen and De Guzman, 1983a,b; Monfort, 1984, 1985; Thanh-Tuyen, 1985, 1990; Pannetier and Buffard-Morel, 1986; Griffis and Litz, 1997; Perera, 2002a,b, 2003, Perera et al. 2006), and date palm *(Phoenixdactylifera)* (Brochard, 1981; Bouguedoura, 1991; Chaibi et al., 2002). There is one report of attempts at ovule culture in coconut (Coconut Research Board, 2002). However no haploid plants were produced from any of these *in vitro* studies. However, there is a single example of a haploid coconut plantlet isolated from a twin seedling (Whitehead and Chapman, 1962), and cytological evidence of a haploid chromosome number (n = 16) observed from a single embryo from the same species. There also a claim that treatment of unpollinated female date palm inflorescences with gibberellic acid induced doubled haploid "apomictic" progeny (Ben Abdallah et al., 2001). However, none of these publications describe an effective method to produce and select spontaneous haploids or doubled haploids or provide teaching relevant to the production of haploid or homozygous material of oil palm.

Oil palm is a perennial monocotyledon, with a long generation period such that breeding of the crop is a very slow process; generally taking approximately 20 years to develop and progeny test a new generation of palms for commercial seed production. There are no reports of breeders producing inbred lines by inbreeding (eight generations of selfing) because this would take a biological minimum of 40 years to achieve because of the time required to make crosses (6 months), process seed (3 months), grow seedlings in nursery (12 months), field plant seedlings - male and female inflorescences will develop after 18 - 24 months, collect pollen & self pollinate palm and harvest bunch (24 - 30 months). Currently, genetic improvement of oil palm is mainly performed by conventional means. Compared to other oil producing crops, which are predominantly annuals, the introduction of novel traits into oil palm is an extremely protracted process; it may require between 12 to 14 years to improve or to introduce a trait into oil palm. In addition to the long generation period, breeding of perennials such as oil palm require large areas for breeding trials and an extensive series of time-consuming backcrosses.

Thus, the lack of progress with oil palm is principally because the breeding system of the crop precludes the simple production of inbred lines. Oil palm is essentially an outbreeding species, but unlike maize in which a male and a female flower are produced on the same plant at the same time, each oil palm plant produces either male or female flowers at any one time, and therefore a tree cannot be easily self-pollinated. Progress in converting oil palm to a hybrid crop, and thereby exploiting the potential hybrid vigour, depends upon the development of a process for the reliable production of homozygous plants.

### Summary of invention

In accordance with a first aspect of the invention, there is provided a method for selecting haploid plants but that may also identify homozygous doubled haploid plants, the method comprising the steps of:
(a) providing a population of seeds, seedlings or plants;
(b) choosing individuals with an atypical phenotype;
(c) assessing the heterozygosity of the chosen individuals using one or more molecular, biochemical or phenotypic markers;
(d) assessing the DNA content of a somatic cell of chosen individuals that are not identified as heterozygous in step (c); and optionally
(e) further assessing the homozygosity of the chosen germinated seedling using multiple molecular markers;
(f) selecting a chosen germinated seed, seedling or plant that is identified as lacking multiple alleles for all markers (i.e. a hemizygous individual for haploids or a completely homozygous individual for doubled haploids) tested on the basis of step (c) or (e) and/or as haploid on the basis of step (d); or highly homozygous on the basis of step (c) or (e) and diploid on the basis of step (d).

Preferably, step (f) is selecting a chosen germinated seedling that is identified as highly homozygous on the basis of step (e) and haploid on the basis of step (d); or step (f) is selecting a chosen germinated seedling that is identified as highly homozygous on the basis of step (e) and diploid on the basis of step (d).

Preferably, the step of using molecular, biochemical or phenotypic markers to assess the heterozygosity of the chosen germinated seedling comprises using between 2 and 40, for example between 10 to 20 microsatellite markers, although similar numbers of markers using one of the many marker systems based on Single Nucleotide Polymorphisms (SNPs) could also be applied (e.g. High Resolution Melt analysis or pyrosequencing).

Preferably, the atypical phenotype is an atypical growth morphology that appears during the germinated seeds or seedling stages. More preferably, the atypical growth morphology is one or more of reduced radicle growth, altered radicle:plumule length ratio, changed radicle:plumule angle, altered colour of radicle or plumule, altered seed shape or size during germination; and altered radicle width:length ratio. The atypical phenotype of a germinated seed may also be the germination of two embryos from a single seed.

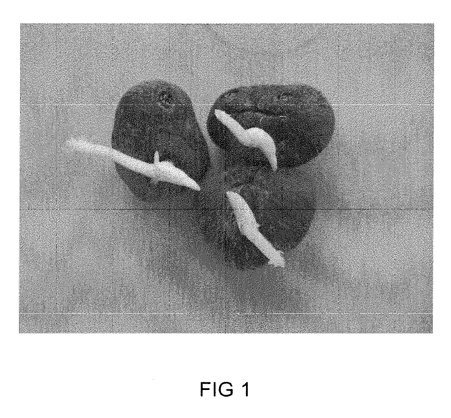

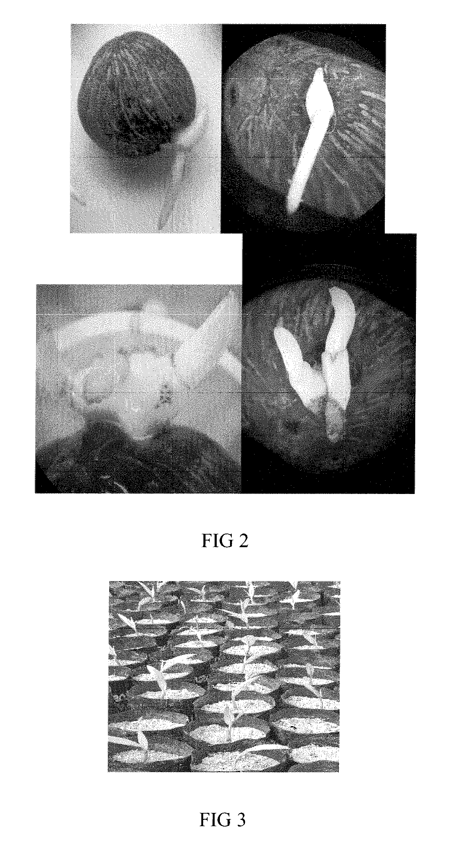

By "desired atypical phenotype", it is meant any aberrant phenotypes exhibited by the haploids and doubled haploids that fall outside the normal phenotypic range expected for non-haploid material (i.e. usually diploid but polyploid for some crops). The confidence limits constituting 'the normal range' may change from species to species but atypical individuals might ordinarily represent less than 1 % of the population screened. For example, in oil palm, candidate haploid seedlings are selected from germinated seed just after the plumule and radicle have developed. Germination will commence after about 10 days incubation in the germination room. Cohorts of germinating oil palm seedlings typically exhibit a fairly synchronous developmental pathway and a reasonably homogenous phenotype (see Figure 1). Abnormal germinated seed may deviate from the characteristic phenotype in one of many ways (see Figure 2) and may include features including those as diverse as reduced radicle growth, altered radicle:plumule length ratio, changed radicle:plumule angle (typically around 180° in normal types), altered colour of radicle or plumule, changes of seed shape or size, changed radicle width:length ratio and germination of two embryos from a single seed (twin seedlings). A key element of novelty here lays in the logical reiterative nature of the selection of features that are used in the definition of atypical. Moreover, as the number of identified haploids increases, ordination approaches are used to identify those traits that are most important in discriminating the atypical set and these are used to redefine the search criteria. This process will progressively improve accuracy of the phenotypic screen as the increasing numbers of haploids enhances statistical power and as uninformative traits are discarded from consideration, and will continue until there are no further increases in haploid frequency.

Preferably, the step of further assessing the homozygosity of the chosen germinating seedling using multiple molecular markers comprises using between 50 and 200, for example between 70 and 120 microsatellite markers. More preferably, this step is performed on a pooled sample of markers. A chosen seedling is identified as highly homozygous if it is homozygous for each molecular marker used.

Preferably, the population of germinated seeds, seedlings or plants comprises at least 1,000,000 individuals. More preferably, the population of germinated seeds, seedlings or plants comprises between 5,000,000 and 20,000,000 individuals. Still more preferably, the population of germinated individuals is a population of plants.

By "providing germinated seeds or seedlings", it is meant to refer to any process whereby the seeds sprout and seedlings begin to grow. In the case of oil palm, it includes any of the two germination techniques used by commercial and plant breeding seed production units: the wet heat method and the dry heat method. The former method is now little used: the whole process may be shorter (95 days against 120 days for dry heat), but some germination will take place during the heating period and so a less uniform set of seedlings will be produced. Oil palm seed is dormant when it is harvested, and under natural conditions germinates sporadically over several years. The critical requirement to break dormancy is to maintain the seed at a raised temperature of 39-40 °C for up to 80 days.

It is understood that the nature of the marker system used or the order in which elements or activities are applied in the above can be adjusted according to circumstances.

The use of markers, preferably co-dominant molecular markers, allows the identification of hemizygous haploids and/or homozygous diploid individuals. Haploid and doubled haploids have only one allele for all loci within their nuclear genomes. Therefore, any individual exhibiting two alleles for any locus can be discarded as a potential haploid/doubled haploid plant. In practice, it may sometimes be preferable to divide this stage into a low-cost pre-screen to discard large numbers of false candidates and a high-resolution genome characterisation (see below) to confirm haploid or doubled haploid status following reference to other stages in the pipeline (e.g. flow cytometry, see below).

Flow cytometry is used for assessing the genome content of plant or animal cells, and can be used to distinguish between diploid and haploid material. By "flow cytometry", it is meant to refer to any method for counting, examining and sorting analyte suspended in a stream of fluid. It allows for simultaneous multiparametric analysis of the desired characteristics of single cells flowing through an optical and/or electronic detection apparatus. In this step, therefore, flow cytometry is applied to the individuals exhibiting an abnormal seedling phenotype and also high levels of homozygosity (identified in steps a and b) to distinguish between the haploids and diploids. Thus, haploid plants are first identified at the completion of this step.

A more comprehensive molecular assessment of genomic heterozygosity is used to provide genetic confirmation of the identity of haploid plants and also identifies individuals that are diploid and are derived from the chromosome doubling of haploid individuals (so-called doubled haploid plants). In both cases, the assessment is based on the fact that haploids and doubled haploids will be completely hemizygous and homozygous respectively. Preferably, microsatellite markers are used for this purpose, although many other marker systems could equally be used. Advantageously, the status of haploids is confirmed and doubled haploid plants identified.

Novelty in this method resides partly in the reiterative nature of the phenotypic screen and the use of the ligation-cloning method to generate large numbers of markers to confirm haploid status but also in the combination of steps to create a method that systematically identifies rare haploids from amongst a large population of seed that are the product of a sexual cross, which has previously been considered impractical.

In accordance with a second aspect of the invention, there is provided a plant selected by the method according with the first aspect of the invention.

In accordance with a third aspect of the invention, there is provided a method for producing a homozygous doubled haploid plant, the method comprising:
(a) selecting a haploid plant using a method according to the first aspect of the invention;
(b) obtaining a doubled haploid plant through spontaneous chromosome doubling; or by doubling the chromosome number by application of an external agent to the haploid plant; or by application of an external agent to a cell or cells isolated from the haploid plant, followed by regeneration of a plant using tissue culture; or by selfing the haploid plant by exploiting the occasional spontaneously doubled chromosome number in male and female reproductive cells.

In accordance with a fourth aspect of the invention, there is provided a method for producing a diploid F₁ hybrid, the method comprising:
(a) selecting at least two homozygous doubled haploid plants using a method according to the first aspect of the invention; or obtaining at least two homozygous doubled haploid plants using a method according to the third or eighth aspect of the invention;
(b) Using two genetically different homozygous doubled haploid plants identified above and sexually crossing these to produce genetically uniform F₁ hybrid offspring.

In accordance with a fifth aspect of the invention, there is provided a plant produced by the method according to the third and fourth aspects of the invention.

In accordance with a sixth aspect of the invention, there is provided a haploid oil palm plant.

In accordance with a seventh aspect of the invention, there is provided a homozygous doubled haploid oil palm plant.

The incorporation of atypical phenotype in the method described above favours the selection of haploid plants over doubled haploid plants since elite examples of the latter may actually exhibit a normal phenotype. For this reason, we also provide a second method that preferentially selects for doubled haploid offspring from the same starting material.

Thus, in accordance a eighth aspect of the invention, there is also provided a method (2) for selecting a homozygous doubled haploid plants, the method comprising:
(a) Identify 20 heterozygous unlinked loci in the maternal parent, preferably using co-dominant molecular markers such as microsatellites or SNP-based markers
(b) Preferably perform a preliminary screen using 1-5 of the selected markers and discard heterozygotes; retain the remainder as candidate doubled haploids
(c) Apply flow cytometry to the retained candidates and discard haploids; retain diploids as potential doubled haploids
(d) Apply the remaining markers to the retained candidates. Individuals that are diploid and homozygous for all 20 loci are deemed doubled haploids (the probability of this occurring by independent assortment is 2²⁰=1/1,048,576)

We are unaware of any study aiming to select doubled haploid, absolute homozygous plants amongst sexual offspring that incorporates prior characterisation of the maternal parent and selection of a fixed number of unlinked heterozygous loci as a basis for subsequent screening. This step allows for standardisation between experiments, genotypes and species since although the markers used may be different, the power of analysis remains the same (in this case a 1/1,048,576 of finding a single false positive homozygous diploid rendered such by independent assortment). This step of the second method is therefore novel, as is the assembly of steps to create the method.

Commercial and government oil palm breeding programmes have not invested in research programmes to identify haploid genotypes despite the inherent value of haploids (or directly doubled haploids) to produce parental true breeding lines and thus F1 hybrids. The discovery of a process to produce haploids in any crop has the potential to transform the rate of breeding progress because well advanced and proven breeding strategies can be adopted from the cereal world crops e.g. rice, wheat, maize etc. Furthermore genetically homogeneous commercial planting material can be produced which further increases value by selecting specific F1 hybrid crosses for particular locations, management practices, or which have certain selected traits.

Instead the oil palm industry for the last thirty years has invested millions of US dollars in the production of oil palm clones by somatic embryogenesis. This is despite the major problems with flowering abnormality which may result in zero bunch yield and the failure of researchers to fully understanding the underlying biological mechanisms which cause flowering abnormality (it is widely assumed to be an 'epigenetic' phenomenon: a modification of gene expression, passing from one cell generation to the next). Despite these difficulties the oil palm industry has remained committed to new investment in oil palm cloning techniques to produce superior genetically uniform plants. It is therefore surprising that until now a process has not been developed to screen for spontaneous haploids and double haploids which would avoid these problems. Previous unpublished attempts have been made by Malaysian oil palm research stations to screen seedlings in the nursery for haploids but with no apparent success.

### Brief Description of Figures

In order that the present invention may be fully understood and readily put into practical effect, there shall now be described by way of non-limitative examples only preferred embodiments of the present invention, the description being with reference to the accompanying illustrative figures.

In the figures:
Figure 1 shows normal seedlings after germination;
Figure 2 shows abnormal seedlings after germination;
Figure 3 shows seedlings after transfer to a nursery house;
Figure 4A shows an example gel used to identify individuals that are homozygous (one band) and heterozygous (two bands) for a selected marker;
Figure 4B is a flow chart showing a hierarchical screen to identify homozygous plants;
Figure 5 shows a representative flow cytometry histogram of samples from a diploid (a) and a haploid (b) genotype;
Figure 6A is a table showing parents of confirmed haploids;
Figure 6B shows electropherograms generated by the bulked-ligation method;
Figure 7A shows haploids and corresponding heterozygous diploid plant;
Figure 7B shows images of a typical diploid heterozygous oil palm (bottom) and two doubled haploids (top) sown on the same day.

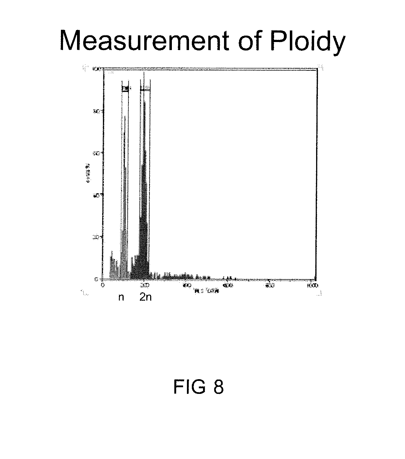

Figure 8 shows the DNA content of haploid and diploid plants as measured using flow cytometry.

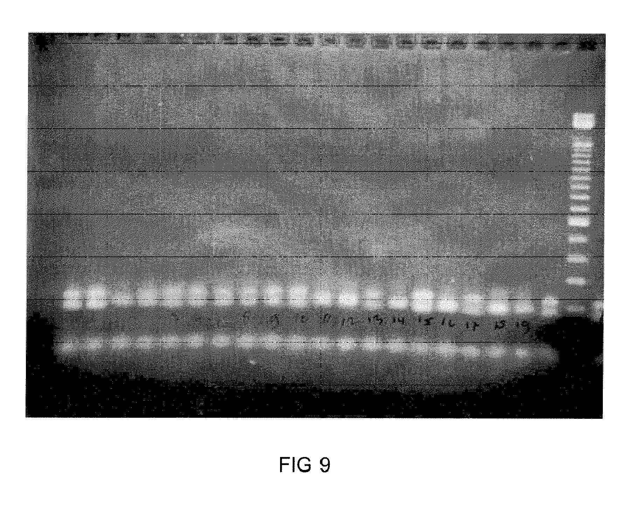

Figure 9 shows a photograph of gel showing use of molecular markers to identify haploids/homozygous diploids (one band) from heterozygous diploids (two bands).
Figure 10 shows confirmed haploid 50-03060260_0002 with first inflorescence two years and seven months after planting (left photograph of inflorescence and right photograph of haploid seedling).

### Detailed Description of the Preferred Embodiments

By "plant", it includes any whole plants, plant organs (e.g., leaves, stems, flowers, roots, etc.), seeds and plant cells (including tissue culture cells) and progeny of same.

By "haploid", it means any cell or plant containing the gametic chromosome number.

By "homozygous", it means any cell or plant containing two identical sets of chromosomes.

By "plantlet", it means any small plant which is not fully grown.

### Example

The following example is presented to further illustrate and explain the present invention and should not be taken as limiting in any regard.

All references herein mentioned are hereby incorporated by reference.

### (1 a) Seed processing

1 The mesocarp was mechanically removed from the seeds and the seeds were air dried for 24 hours at ambient temperature and then for 24 hours in an air-conditioned room at 25 °C to a seed moisture content of 15-18%. The seeds were then stored, usually for one to three months in an air-conditioned room (25 °C) in plastic bags or trays (although it is possible to store seeds for up to one year in this way).
2 The seeds were soaked for three days to increase their moisture content to 18 - 20 % and then heat treated in plastic bags or trays for 40 to 60 days at 38 - 40 °C.
3 After heating, the seeds were soaked for five days to raise their moisture content to >22% and then dried at ambient temperature for approximately four hours
4 The seed were transferred to a germination room where under ambient temperatures germination usually starts after 7 to 10 days and continues for two to three months.

### (1b) Morphological screen

There were two large-scale morphological screens of seedlings for morphological off-types. The first consisted of 10,900,000 germinated seeds, of which 3,854 were identified as being morphologically deviant (3,801) or twin-seeded (53), with the remaining individuals all being deemed 'normal' (see Figures 1 and 2 for examples of both types). Thus, in this instance 99.96% of seeds evaluated were classified as exhibiting a normal phenotype and 0.035% being aberrant. In the second screen, approximately 10,000,000 commercial seedlings were screened, together with approximately 1,000,000 seedlings taken from breeding experiments. This trial generated 5,704 morphological candidates, of which 5,601 were phenotypically abnormal and 103 were twin-seeded. In this screen, therefore, 99.95% of seedlings were classed as normal and 0.05% as aberrant prior to transfer to the nursery house (Figure 3).

### (1c) Molecular pre-screen

### Molecular pre -screen to exclude heterozygous individuals.

The protocol applied to perform a molecular prescreen of seedlings showing abnormal phenotypes to discard heterozygotes comprised the following stages:
*1. DNA extraction*
*2. Amplification of microsatellite mar kers by PCR*
*3. Separation of PCR products by agarose gel electrophoresis*
*4. Scoring of results to discard individuals with one or more heterozygous loci*

Each stage is described below:

### 1. DNA extraction

Around 0.5 cm of the radicle (around 50 mg) was removed from the seedling and used to extract DNA using the Qiagen 96 DNeasy extraction kit according to the manufacturer's instructions as described below, although other systems for DNA extraction could also be used.

### A. PREPARATION

1. For new kits, add 100% ethanol to AP3/E buffer and AW buffer
2. Set water bath to 65°C
3. Preheat AE and AP1 buffer to 65°C
4. If AP1 buffer has a cloudy appearance, heat to 65oC and shake until the solution becomes clear

### B. PROTOCOL

1. Add 50 mg plant material into each tube in two collection microtube racks. Retain the clear cover.
2. Add one tungsten carbide bead into each microtube.
3. Prepare the lysis solution: (400 µl AP1+ 1 µl RNAse + 1 µl Reagent DX)/reaction plus 15% of each component.
4. Disrupt the sample using MM 300, 30 Hz for 1.5 minutes.
5. Pulse centrifuge to 3000 rpm.
6. Remove and discard caps, add 130 µl AP2 buffer into each collection microtube.
7. Close the microtubes with new caps. Place a clear cover (from step 1) over the 96 well plate. Shake the plate vigorously for 15s. Pulse centrifuge to 3000 rpm.
8. Incubate the racks for 10 min at -20°C.
9. Remove and discard the caps. Transfer 400 µl of each supernatant to new plate of collection microtubes (provided). Do not transfer pellet and floating particles. Hold the strips and use the lowest pipette speed. Recover the tungsten beads.
10. Add 1.5 volume (typically 600 µl) of AP3/E buffer.
11. Close the microtubes with new caps and mix vigorously.
12. Pulse centrifuge (3000rpm) to collect solution).
13. Place 96 well plates on top of S-Blocks provided.
14. Transfer 1 ml of sample into each well of the 96 well plate.
15. Seal with Airpore Tape sheet and centrifuge for 4 min at 6000 rpm.
16. Add 800 µl of Buffer AW to each sample.
17. Centrifuge for 15 min at 6000 rpm.
18. Add 100 µl of buffer AE to each sample and seal with new AirPore sheets.
19. Incubate for 1 min at room temperature (15-25°C).
20. Centrifuge for 2 min at 6000 rpm.

### 2. Amplification of microsatellite markers by PCR

### Primers

The following microsatellite markers were used:

| | Forward primer | Reverse primer |
|---|---|---|
| Marker 1 | GAGATTACAAAGTCCAAACC | TCAAAATTAAGAAAGTATGC |
| Marker 2 | ACGCATGCAGCTAGCTTTTC | CGCGTGAAAGATATGAATCAAC |
| Marker 3 | CACGCACGCAGTTTATTCTT | GGATGTATGCTTTACCTCCGAAT |
| Marker 4 | CCCCTTTTGCTTCCCTATTT | CTCCTTTTCCCCATCACAGA |
| Marker 5 | GACACAAGCAAAAACAAAAGCA | ATTCTGAAAGGAGGGGGAAA |
| Marker 6 | ATATGTGTGGGTGTGCGTGT | TGCCTCTGGTTGTTAGTCTGG |
| Marker 7 | TCTCTCTCTCTCTCTCTATGTGTGTGT | TGGCAATCAGCACACATTCT |
| Marker 8 | GCAGCTCTTTCCACACCTCT | TGTGGTCTCCTGAGGAAGATG |
| Marker 9 | TTTTCCCCATCACAGAATTG | CCCCTTTTGCTTCCCTATTT |
| Marker 10 | TAGCCGCACTCCCACGAAGC | CCAGAATCATCAGACTCGGACAG |
| Marker 11 | AGCTCTCATGCAAGTAAC | TTCAACATACCGTCTGTA |
| Marker 12 | CCTTCAAGCAAAGATACC | GGCACCAAACACAGTAA |
| Marker 13 | GTAGCTTGAACCTGAAA | AGAACCACCGGAGTTAC |
| Marker 14 | GCTCGTTTTTGTTTAGGTGA | TTTTCTCCATAGTCCGTTAC |
| Marker 15 | CCTCGGGTTATCCTTTTTACC | TGGCTGGCTTCGGTCTTAG |

| | | |
|---|---|---|
| Note: Markers 10-15 were obtained from Billotte et al (2005) | | |

### Reaction Mixtures

In all cases, 10 µl a PCR reaction mixture contained the following reagents; 1.0 µl of 10x PCR buffer (Bioline), 0.3 µl MgCl₂ (10 mM), 0.4µl dNTPs (10 mM of each), 0.2 µl of each primer pair (10 µM), 1-5 ng of DNA (extracted as above) and 1 U of *Taq* DNA polymerase (5 U µl⁻¹ Bioline).

### PCR conditions

The following conditions were used for the Polymerase Chain Reaction for all microsatellite markers: an initial 94°C denaturing step for 2 min followed by 35 cycles of; 94°C for 30 sec, 52°C for 30 sec and 72°C for 45 sec, with a final extension step of 72°C for 7 min.

### 3. Separation of PCR products by agarose gel electrophoresis

Agarose gel electrophoresis and ethidium bromide staining were routinely used to fractionate and visualise products generated by microsatellite PCR.

### (1) Reagents

| | |
|---|---|
| *TBE running buffer :* | 0.089 M Tris base, 0.089 M boric acid (pH 8.3) and 2 mM Na₂EDTA |
| Loading buffer: | 0.23% (w/v) bromophenol blue 60 mM EDTA 40% (w/v) sucrose |
| *Ethidium bromide stain :* | 1 % (w/v) ethidium bromide |
| *Ladder 100 bp* (Gibco Life Science BRL) | |

### (2) Gel preparation and loading

1.0 - 1.5 % (w/v) agarose (was prepared in 1 x TBE buffer and subjected to heating in a microwave (700W) for 2 x 1 min at full power to create a gel solution. The gel solution was cooled to approximately 55°C prior to the addition of ethidium bromide (3.5 µl per 100 ml gel). The ends of a suitable gel tray rig (midi-gel tray for 100 ml gels, maxi-gel tray for 250 ml gels) were sealed with masking tape and an appropriate number and type of combs placed in position. Combs with 16 x 20 µl wells were most often employed. The gel solution was carefully poured into the prepared tray and allowed to cool for at least 20 min. Combs and tape were then removed and the gel tray submerged into a tank containing 1 x TBE buffer.

Generally, 5 µl of sample were mixed with 2 µl of bromophenol blue buffer prior to loading. The loading buffer serves two functions: first, it increases the specific gravity of the sample thereby preventing diffusion of DNA from the top of the well into the surrounding buffer, and second, it indicates the progress of product as they migrate through the gel by electrophoresis (the blue dye migrates at approximately the same position as DNA fragments 200 bp in length). To estimate the size of the amplicons, 4µl of 100 bp Gibco's ladder (Gibco Life Science BRL) were loaded together with the analysed samples.

Electrophoresis of mid-gels (100 ml) was performed at 120 Volts in 1X TBE buffer for approximately 1 h. Following electrophoresis, gels were removed from the rig and post-stained in 5 mg/l aqueous ethidium bromide solution for 40 min, destained in distilled water for 2 min and then viewed under Ultra Violet Illumination using a UVP Bio-Doc-system. Images of the gels were captured by the UVP Bio-Doc system as jpeg format and used for scoring.

### 4. Scoring of results to discard individu als with one or more heterozygous loci

PCR products generated by each microsatellite-genotype combination were evaluated for the presence of one or two distinct bands after fractionation by agarose gel electrophoresis (stages 1-3 above). Any genotype that yielded two products for any of the microsatellite loci was deemed to be heterozygous and so discarded as a possible candidate haploid or doubled haploid plant. The remaining individuals were sent forward to step (d) of the pipeline (flow cytometry)

### Results

There were over 2000 phenotypically abnormal seedlings identified from the two morphological screens described above that were randomly selected for the molecular screen. In addition, there were a further 150 individuals with the normal phenotype. There were also 24 diploid tenera clones used as controls (all diploid heterozygotes).

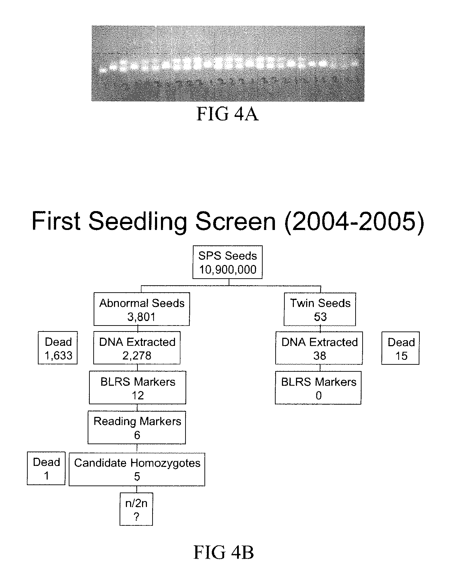

When these were screened using up to 15 of microsatellite markers (1-15), 117 genotypes (see table in flow cytometry section for identification codes) exhibited a single allele for all loci (an example is shown in Figure 4, using marker 09) and so were deemed to be highly homozygous.

Accordingly, these individuals were considered as candidate haploids/doubled haploids and progressed to step e (flow cytometry).

Figure 4 shows Band profiles generated by marker 09 across 25 oil palm genotypes. Individuals showing two alleles (marked '2') were discarded from the screen.

### (1d) Assessment of nuclear genome content by flow cytometry

### Flow Cytometry

Individuals identified as morphologically abnormal and highly homozygous (stages b and c) were subjected to flow cytometry to establish their ploidy level using the following protocol.

### Sample preparation

The cell nuclei were isolated from fresh plant material (leaves or roots), by chopping the plant material (a few cm² /20-50 mg)) with a sharp razor blade in an ice-cold buffer, in a plastic petri dish. The DNA buffer (stored at 4 °C) is based on:
Arumuganathan, K. and Earle, E.D. Estimation of Nuclear DNA Content of Plants by Flow Cytometry. Plant Molecular Biology Reporter, Vol 9(3) 1991, Pages 229-233.
5 mM Hepes
10 mM Magnesium sulphate heptahydrate
50 mM Potasium chloride
0,2 % Triton X-1 00
2% DTT (Dithiothreitol)
2 mg/litre DAPI
pH 8

DAPI, a fluorescent dye that selectively binds to form a complex with doublestranded DNA and give a product that fluoresces at 465 nm, was introduced to the solution. DAPI has specific DNA-binding properties, with preference for adenine-thymine (AT)-rich sequences. After chopping, the buffer (ca. 2 ml.), containing cell constituents and large tissue remnants, is passed through a nylon filter of 40 micrometer mesh. This method will produce thousands of nuclei from a leaf piece of a few cm².

The solution containing stained nuclei was passed through the flow cytometer.

Controls are required of known ploidy (DNA content) as reference - for oil palm, tissue from diploid tenera palms were used because the shell thickness must be heterozygous and therefore the palm cannot be haploid.

The fluorescence of the stained nuclei, passing through the focus of a light beam from a high-pressure mercury lamp, was measured by a photomultiplier and converted into voltage pulses.

These voltage pulses were electronically processed to yield integral and peak signals that can be processed by a computer. When the samples are run with the appropriate filter-settings for excitation and emission, DNA histograms can be produced.

### Material

Flow cytometer: CyFlow ML (Partec GmbH, Otto Hahnstrasse 32, D-4400 Münster, Germany) with a high pressure mercury lamp, OSRAM HBO 100 long life. Objective: 40 x N.A. 0,8 air (Partec)
Filter combination with DAPI:
Heat protection filter KG-1
Excitation-filters: UG-1 and BG-38.
Dichroic mirrors: TK 420 and TK 560.
Emission-filter: GG 435

Software: Flomax version 2.4 d (Partec)

### Results

Of the 117 genotypes identified as highly homozygous in step c, 83 were identified as haploid by flow cytometry, with remaining 34 individuals being diploid (see Table 1 below)

**Table 1. Ploidy level (x or 2x;haploid or diploid) of homozygous clones identified using markers 1-15**

| Candidate | DNA sample code | Σ Primers Used in screening | Flow cytometry Result |
|---|---|---|---|
| 50-Mix5-7 | 11260406301 | 9 Primer | x |
| 50-03060367C | 07280501801 | 15 Primer | x |
| 50-03060260C-2 | 07280501901 | 15 Primer | x |
| 53-03080954C-2 | 09270500101 | 10 Primer | x |
| 53-03090761C-5 | 09280504501 | 10 Primer | x |
| BATCH 51;03060318C;1 | 060728_0010_01_a | 15 Primer | x |
| BATCH 53;03090761C;5 | 060728_0018_01_a | 15 Primer | x |
| 0623/172;05095508C;1 | 060728_0021_01_a | 15 Primer | x |
| BATCH 50;03060260C;2 | 060728_0027_01_a | 15 Primer | x |
| 0611/32;05050248C;1 | 060728_0032_01_a | 15 Primer | x |
| 0611/16;05050228C;1 | 060728_0034_01_a | 15 Primer | x |
| BATCH 53;03080954C;2 | 060728_0035_01_a | 15 Primer | x |
| 06 412;04059061B;3 | 060728_0050_01_a | 14 Primer | 2x |
| 0628/152;05100720C;1 | 060729_0021_01_a | 15 Primer | x |
| 0628/185;05100351C;1 | 060729_0063_01_a | 15 Primer | x |
| BATCH 51;03060626C;1 | 060729_0127 _02_a | 15 Primer | x |
| BATCH 67;0409034MC;2 | 060729_0130_02_a | 14 Primer | 2x |
| BATCH 67;0409034MC;4 | 060729_0131_02_a | 15 Primer | 2x |
| BATCH 67;0409034MC;15 | 060729_0132_02_a | 15 Primer | 2x |
| BATCH 65;0409034MC;7 | 060729_0134_02_a | 15 Primer | 2x |
| BATCH 65;0409034MC;35 | 060729_0138_02_a | 15 Primer | 2x |
| BATCH 65;0409034MC;56 | 060729_0139_02_a | 15 Primer | 2x |
| BATCH 65;0409034MC;50 | 060729_0141_02_a | 15 Primer | 2x |
| BATCH 65;0409034MC;47 | 060729_0142_02_a | 15 Primer | 2x |
| 0628/53;05090595C;1 | 060731_0043_01_a | 15 Primer | x |
| 0627/125;05090717C;2 | 060731_0065_01_a | 15 Primer | x |
| 0627/12;05080220C;1 | 060731_0080_01_a | 15 Primer | x |
| 0627/6;05080095C;1 | 060731_0086_01_a | 14 Primer | x |
| 0631 /Normal; 05039033B;31 | 060731_0265_01_a | 14 Primer | x |
| 64-0409021 MC-34 | 02130604301 | 15 Primer | 2x |
| 64-0410040MC-1 | 02130604801 | 15 Primer | 2x |
| 51-03060626C | 02130605301 | 15 primer | x |
| 64-0410040MC-20 | 02140600401 | 15 primer | 2x |
| 64-0410040MC-16 | 02140600801 | 15 primer | 2x |
| 65-0409021MC-2 | 02140601001 | 15 primer | 2x |
| 06 412B-04059061B-3 | 02170605501 | 15 Primer | 2x |
| 06 412B-04129091B | 02170605801 | 15 Primer | 2x |
| 0550-15/05010827C | 02200602401 | 15 Primer | x |
| 0550-17/05010442C-1 | 02200602601 | 15 Primer | x |
| 0550-23/05020059C | 02200603101 | 15 Primer | x |
| 0550-33/05020568C | 02200603401 | 15 Primer | x |
| 0550-36/05020420C-2 | 02200603701 | 15 Primer | x |
| 0550-40/05010880C | 02200607501 | 14 Primer | x |
| 0551-36/05020511C | 02200607601 | 15 Primer | x |
| 0551-32/05020361C-1 | 02210600401 | 15 Primer | x |
| 0552-4/05010836C-2 | 02210600901 | 15 Primer | x |
| 0552-38/05020501C | 02210603101 | 14 Primer | x |
| 0552-39/05020415C | 02210603201 | 15 Primer | x |
| 0552-31/05020858C | 02210603701 | 15 Primer | x |
| 0552-91/05020375C | 02210603901 | 15 Primer | x |
| 0552-111/05020626C | 02210607201 | 15 Primer | x |
| 0552-128/05020558C-1 | 02210607701 | 15 Primer | x |
| 0601-35/05020946C | 02210608201 | 15 Primer | x |
| 0601-42/05030201C-6 | 02210609501 | 15 Primer | x |
| 0601-51/05030224C-2 | 02220600201 | 15 Primer | x |
| 0607-21/05040317C-3 | 02220601801 | 14 Primer | x |
| 0606-32/05040240C | 02220606201 | 13 Primer | x |
| 0601-77/05020961C | 02230600701 | 15 Primer | x |
| 0601-62/05030147C | 02230601401 | 15 Primer | x |
| 0601-54/05030462C | 02230601901 | 15 Primer | x |
| 0551-21/05020271C-1 | 02200605801 | 14 Primer | x |
| 0601-9/05020843C-2 | 02230603101 | 15 Primer | x |
| 0602-17/05020631C-1 | 02230605501 | 16Primer | x |
| 0607-111/05040970C-1 | 03010600201 | 15 primer | x |
| 0607-81/05040578C-1 | 03010600501 | 15 primer | x |
| 0607-73/05040573C-1 | 03010605101 | 15 Primer | x |
| 0607-89/05040748C-3 | 03010605501 | 15 primer | x |
| 0607-102/05050016C-2 | 03010606601 | 15 primer | x |
| 0608-15/05040519C-3 | 03010606901 | 15 primer | x |
| 0608-45/05041003C-1 | 03150603401 | 15 Primer | x |
| 0610-60/05041024C-2 | 03150604401 | 15 primer | x |
| 0610-124/05055039C-1 | 03150604601 | 15 primer | x |
| 0609-54/05050089C-2 | 03150604701 | 15 primer | x |
| 0610-41/05050352C-1 | 03150606701 | 15 primer | x |
| 0609-58/05050255C-1 | 03220600201 | 15 primer | x |
| 0610-82/05050099C-2 | 03220601401 | 15 primer | x |
| 0610-77/05050353C-1 | 03220602701 | 15 primer | x |
| 0610-121/05055090C-1 | 03220603301 | 15 Primer | x |
| 0610-81/05050099C-1 | 03220605901 | 15 primer | x |
| 0609-100/05055311C-1 | 03290600301 | 15 primer | x |
| 0610-11/05040938C-1 | 03290601101 | 15 primer | x |
| 0610-68/05050376C-3 | 03290602001 | 15 primer | x |
| 0610-58/05050344C-1 | 03290602201 | 15 primer | x |
| 0610-73/05050594C-3 | 03290603301 | 15 primer | x |
| 0611-84/05050714C-4 | 03290605001 | 15 primer | x |
| 0611-70/05050223C-1 | 03290606701 | 15 primer | x |
| 0611-73/05050351C-1 | 03290608001 | 15 Primer | x |
| 0610-67/05050376C-2 | 04050600501 | 15 primer | x |
| 0610-40/05050102C-2 | 04050600901 | 15 primer | x |
| 0611-99/05050544C-1 | 04050602601 | 15 primer | x |
| 0611-110/05055011C-1 | 04050603601 | 15 primer | x |
| 0612-2/05050017C-1 | 04050609101 | 15 primer | x |
| 0612-70/05050530C-1 | 04050609201 | 15 primer | x |
| 0612-76/05050512C-1 | 04050610301 | 15 Primer | x |
| 0611-109/05055144C-1 | 04120600101 | 15 Primer | x |
| 0611-31/05050220C-1 | 04120600601 | 15 Primer | x |
| 0611-38/05050284C-4 | 04120600901 | 15 Primer | x |
| 0611-40/05050171C-1 | 04120601101 | 14 Primer | x |
| 0612-80/05050713C-1 | 04120603101 | 15 Primer | x |
| 65-0409034MC-66 | 060829_0001_02_a | 15 Primer | 2x |
| 65-0409034MC-68 | 060829_0002_02_a | 15 Primer | 2x |
| 65-0409034MC-72 | 060829_0003_02_a | 14 Primer | 2x |
| 65-0409034MC-111 | 060829_0005_02_a | 15 Primer | 2x |
| 65-0409034MC-94 | 060829_0011_02_a | 14 Primer | 2x |
| 65-0409034MC-120 | 060829_0012_02_a | 15 Primer | 2x |
| 65-0409034MC-144 | 060829_0013_02_a | 15 Primer | 2x |
| 65-0409034MC-133 | 060829_0015_02_a | 15 Primer | 2x |
| 65-0409034MC-187 | 060829_0020_02_a | 15 Primer | 2x |
| 65-0409034MC-193 | 060829_0021_02_a | 14 Primer | 2x |
| 65-0409034MC-199 | 060829_0023_02_a | 15 Primer | 2x |
| 65-0409034MC-135 | 060829_0025_02_a | 15 Primer | 2x |
| 65-0409034MC-114 | 060829_0026_02_a | 13 Primer | 2x |
| 65-0409034MC-147 | 060829_0027_02_a | 15 Primer | 2x |
| 65-0409034MC-36 B | 060829_0030_02_a | 15 Primer | 2x |
| 65-0409034MC-39 A | 060829_0031_02_a | 15 Primer | 2x |
| 65-0409034MC-73 A | 060829_0034_02_a | 15 Primer | 2x |
| 65-0409034MC-71 A | 060829_0035_02_a | 14 Primer | 2x |

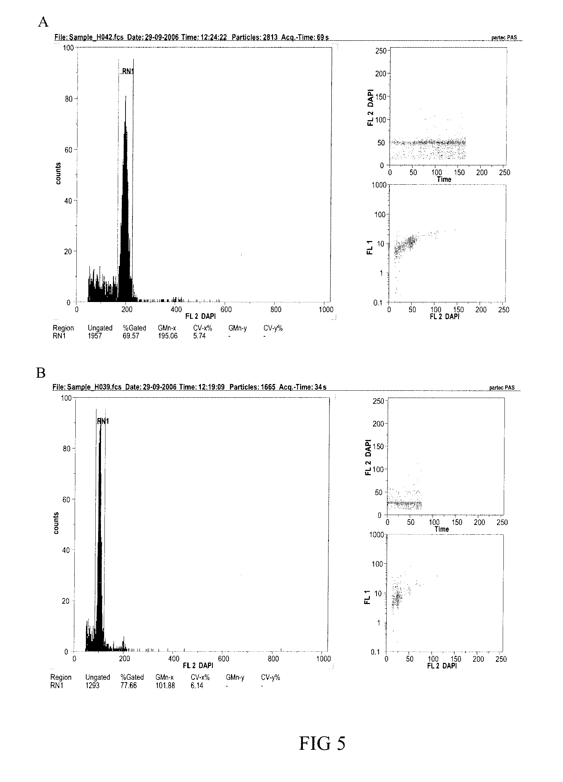

Example histograms are shown in Figure 5.

### (1 e) Genome characterization

Genome characterisation was used for two purposes. First, to confirm the lack of heterozygosity among plants identified as haploids by the morphological assessment, molecular screen and by flow cytometry. Second, to identify doubled haploids on the basis of being diploid and lacking any detectable heterozygosity. The method used to assess both sets of plants was identical and is described below.

### Marker strategy

Genome characterisation (for heterozygosity) was performed using 96 microsatellite loci. Rather than screening all primers against all candidate samples using labelled primers, a pooling strategy (as proposed by Cryer et al., 2005) was adopted that avoids the need for large numbers of expensive, labelled SSR primers. The method involves amplifying each microsatellite locus for all haploid candidates using unlabelled primers, bulking and ligating the products together into a vector, and then performing a second amplification using a fluorescently labelled vector primer to expose allelic forms. The number of alleles at each locus for all individuals could then be assessed by fractionation on the capillary sequencer. Validity of the results obtained by this method was verified by comparing profiles generated using the pooled strategy with those obtained on 10 representative samples (diploid and haploid) using a subset of 24 labelled microsatellite markers fractionated and detected by conventional capillary electrophoresis.

### Genome characterisation using bulk ligation of PCR products

The first step in the screen involved amplifying 12 candidate samples; using 96 microsatellite markers listed in Table 2 (below). For all reactions, the 10 µl microsatellite reaction mixture contained the following reagents; 1.0 µl of 10x PCR buffer (Bioline), 0.3 µl MgCl₂ (10 mM), 0.4 µl dNTPs (10 mM of each), 2.0 µl of each primer pair (1 µM), 1-5 ng of DNA (extracted at BLRS) and 1 U of *Taq* DNA polymerase (5 U µl⁻¹ Bioline). The thermal cycler was programmed with an initial 94°C denaturing step for 2 min followed by 35 cycles of; 94°C for 30 s, 52°C for 30 sec and 72°C for 45 sec, with a final extension step of 72°C for 7 min. PCR products were assessed for size by electrophoresis through a 1% w/v agarose gel for 30 min at 120 V.

**Table 2. Microsatellite markers used for bulk ligation screen (Billote et al. 2005).**

| Marker | Forward primer | Reverse Primer |
|---|---|---|
| MARKER 16 | GACCTTTGTCAGCATACTTGGT GTG | GCAGGCCTGAAATCCCAAAT |
| MARKER 17 | ATGCATGTGATTTTATTAGGTGA GA | CGACCCTCAGTCAATCAGTAAG |
| MARKER 18 | AAGCTAGCGACCTATGATTTTAG | AAACAAGTAATGTGCATAACCT ATTC |
| MARKER 19 | CCCACCACCCCTAGCTTCTC | ACCCCGGTCCAAATAAAATC |
| MARKER 20 | AGAGAGAGAGAGTGCGTATG | GTCCCTGTGGCTGCTGTTTC |
| MARKER 21 | GGGTAGCAAACCTTGTATTA | ACTTCCATTGTCTCATTATTCT |
| MARKER 22 | CGAGGCCCAAAAACATTCAC | GGTCCCGATCCCGTCTACTG |
| MARKER 23 | TTGCGGCCCATCGTAATC | TCCCTGCAGTGTCCCTCTTT |
| MARKER 24 | AGGGAATTGGAAGAAAAGAAAG | TCCTGAGCTGGGGTGGTC |
| MARKER 25 | AGCAAGAGCAAGAGCAGAACT | CTTGGGGGCTTCGCTATC |
| MARKER 26 | TAGCCATGCCGCCACCACTT | CAATCCATTAGCGTGCCCTTCT |
| MARKER 27 | CTTACCCCGCCTCCTCTCCT | CGAAATGCCCTTCCTTTACACT A |
| MARKER 28 | CCTTATATCGCACGGGTTCC | TTCTTGGGGTCTCGCTACGG |
| MARKER 29 | GCAAGATGCAATGGAGTTCA | CAAACCGCAGCAAGTCAGA |
| MARKER 30 | GCAAAATTCAAAGAAAACTTA | CTGACAGTGCAGAAAATGTTAT AGT |
| MARKER 31 | CGTTCATCCCACCACCTTTC | GCTGCGAGGCCACTGATAC |
| MARKER 32 | GAATGTGGCTGTAAATGCTGAG TG | AAGCCGCATGGACAACTCTAGT AA |
| MARKER 33 | ACATTCCCTCTATTATTCTCAC | GTTTTGTTTGGTATGCTTGT |
| MARKER 34 | AAGCCAACTTCACAGATATGTTG | ATGAGCCTAACAAAGCACATTC ATTAA |
| MARKER 35 | AGTGAGGTATGGTTGATTAGGA | TATTGATAGCATTTGGGATTAG |
| MARKER 36 | CTCCGATGGTCAAGTCAGA | AAATGGGGAAGGCAATAGTG |
| MARKER 37 | GCCGTTCAAGTCAATTAGAC | TTTGGGAGCAAGCATTATCA |
| MARKER 38 | TGCTTCTTGTCCTTGATACA | CCACGTCTACGAAATGATAA |
| MARKER 39 | CACCACATGAAGCAAGCAGT | CCTACCACAACCCCAGTCTC |
| MARKER 40 | TTTTATTTTCCCTCTCTTTTGA | ATTGCGTCTCTTTCCATTGA |
| MARKER 41 | CATATGGCGCACAGGCAC | GCAATACAAGAGCACCCAAAT |
| MARKER 42 | AGTTGGTTTGCTGATTTG | TGTTGCTTCTTTGATTTTC |
| MARKER 43 | GCTGAAGATGAAATTGATGTA | TTCAGGTCCACTTTCATTTA |
| MARKER 44 | ATGACCTAAAAATAAAATCTCAT | ACAGATCATGCTTGCTCACA |
| MARKER 45 | GGTGCAAGAGAGGAGGAATG | TTTGGTAGTCGGGCGTTTTA |
| MARKER 46 | GTTTGGCTTTGGACATG | TCCATCACAGGAGGTATAG |
| MARKER 47 | TGTTTTGTTTCGTGCATGTG | GGCTGACATGCAACACTAAC |
| MARKER 48 | CGGTTTTGTCGCATCTATG | GTCGTCAGGGAACAACAGT |
| MARKER 49 | CAATCATTGGCGAGAGA | CGTCACCTTTCAGGATATG |
| MARKER 50 | GAGCATGACGCAAACAAAGG | GCAACATGTTTGATGCATTAAT AGTC |
| MARKER 51 | TCCAAGTAGCAAATGATGAC | TGCCCTGAAACCCTTGA |
| MARKER 52 | GAAGGGGCATTGGATTT | TACCTATTACAGCGAGAGTG |
| MARKER 53 | AACACTCCAGAAGCCAGGTC | GGTTTAGGTATTGGAACTGATA GAC |
| MARKER 54 | GATCCCAATGGTAAAGACT | AAGCCTCAAAAGAAGACC |
| MARKER 55 | TGTGGTTTGAGGCATCTTCT | GCCCACCAAAAGAAAGTAGT |
| MARKER 56 | TAGCCGCACTCCCACGAAGC | CCAGAATCATCAGACTCGGACA G |
| MARKER 57 | TCAAAGAGCCGCACAACAAG | ACTTTGCTGCTTGGTGACTTA |
| MARKER 58 | GGGGATGAGTTTGTTTGTTC | CCTGCTTGGCGAGATGA |
| MARKER 59 | TCTAATGCTCCCAAGGTACA | GGCTTGGTCCACGATCTT |
| MARKER 60 | AGCTCTCATGCAAGTAAC | TTCAACATACCGTCTGTA |
| MARKER 61 | TCCTCACTGCTCCTCTAATC | ACTCCCTATGGACCTTAGTC |
| MARKER 62 | AGGGAGGCGAACGAGAAACA | CGACTGCTGATGGGGAAGAG |
| MARKER 63 | CTACGGACTCACACCTATAT | ATGGTTCATCAATGAGATC |
| MARKER 64 | GTGAGCGATTGAGGGGTGTG | GGGGCTTGATTGAGTATTTCCA |
| MARKER 65 | AGGGCAAGTCATGTTTC | TATAAGGGCGAGGTATT |
| MARKER 66 | GAAGCCTGAGACCGCATAGA | TTCGGTGATGAAGATTGAAG |
| MARKER 67 | TTTCTTATGGCAATCACACG | GGAGGGCAGGAACAAAAAGT |
| MARKER 68 | GTTTATCATTTTGGGGTCAG | CGGTGTCCCTCAGGATGTA |
| MARKER 69 | CATGCACGTAAAGAAAGTGT | CCAAATGCACCCTAAGA |
| MARKER 70 | AATCCAAGTGGCCTACAG | CATGGCTTTGCTCAGTCA |
| MARKER 71 | TGTAGGTGGTGGTTAGG | TGTCAGACCCACCATTA |
| MARKER 72 | AGCAAGACACCATGTAGTC | GACACGTGGGATCTAGAC |
| MARKER 73 | AAAAGCCGATAGTGGGAACA | ATGCTGAGAGGTGGAAAATAG AG |
| MARKER 74 | GTCCATGTGCATAAGAGAG | CTCTTGGCATTTCAGATAC |
| MARKER 75 | AGCCAATGAAGGATAAAGG | CAAGCTAAAACCCCTAATC |
| MARKER 76 | CAATTCCAGCGTCACTATAG | AGTGGCAGTGGAAAAACAGT |
| MARKER 77 | GGGCTTTCATTTTCCACTAT | GCTCAACCTCATCCACAC |
| MARKER 78 | GACAGCTCGTGATGTAGA | GTTCTTGGCCGCTATAT |
| MARKER 79 | ACTTGTAAACCCTCTTCTCA | GTTTCATTACTTGGCTTCTG |
| MARKER 80 | CCTTCAAGCAAAGATACC | GGCACCAAACACAGTAA |
| MARKER 81 | CCACTGCTTCAAATTTACTAG | GCGTCCAAAACATAAATCAC |
| MARKER 82 | GGGAGAGGAAAAAATAGAG | CCTCCCTGAGACTGAGAAG |
| MARKER 83 | AGCAGGGCAAGAGCAATACT | TTCAGCAGCAGGAAACATC |
| MARKER 84 | GCCTATCCCCTGAACTATCT | TGCACATACCAGCAACAGAG |
| MARKER 85 | CATCAGAGCCTTCAAACTAC | AGCCTGAATTGCCTCTC |
| MARKER 86 | ATTCATTGCCATTCCCTTCA | TTGTCCCCTCTGTTCACTCA |
| MARKER 87 | ATTGCAGAGATGATGAGAAG | GAGATGCTGACAATGGTAGA |
| MARKER 88 | TCTCCCAAATCACTAGAC | ATCTGCAAGGCATATTC |
| MARKER 89 | ACGTTTTGGCAACTCTC | ACTCCCCTCTTTGACAT |
| MARKER 90 | TCCACTCTGGCAACTCC | AAGGATGGGCTTTGTAGT |
| MARKER 91 | TTTAGAGGACAAGGAGATAAG | CGACCGTGTCAAGAGTG |
| MARKER 92 | AGCAAAATGGCAAAGGAGAG | GGTGTGTGCTATGGAAGATCAT AGT |
| MARKER 93 | GTAGCTTGAACCTGAAA | AGAACCACCGGAGTTAC |
| MARKER 94 | AAGCCACCAGGATCATC | GTCATTGCCACCTCTAACT |
| MARKER 95 | TTACTTGCTAAGCTCTCTAGC | TGGCTGTTTAATCTGTCTG |
| MARKER 96 | TCTATATTTGGTTGGCTTGA | ACTCATTTCAATCTCAGTGTC |
| MARKER 97 | TGCTACGTGCTGAAATA | ATTTCAGGTTCGCTTCA |
| MARKER 98 | CCTCCACTTCTCTTCATCTT | CTTCCTCAAGCTCAAACAAT |
| MARKER 99 | GATGTTGCCGCTGTTTG | CATCCCATTTCCCTCTT |
| MARKER 100 | ATGCTCCACCAAGTTTA | CACATCCTAGCATCATTG |
| MARKER 101 | AAGCAATATAGGTTCAGTTC | TCATTTTCTAATTCCAAACAAG |
| MARKER 102 | GCTCGTTTTTGTTTAGGTGA | TTTTCTCCATAGTCCGTTAC |
| MARKER 103 | CAGCACACAAATGACAT | CACCTTTCCTTTTTGTC |
| MARKER 104 | CCTATTCCTTACCTTTCTGT | GACTTACTATCTTGGCTCAC |
| MARKER 105 | CCTTGCATTCCACTATT | AGTTCTCAAGCCTCACA |
| MARKER 106 | CCTCCTTTGGAATTATG | GTGTTTGATGGGACATACA |
| MARKER 107 | ATTGGAGAGCACTTGGATAG | TTCTCTTCCTTCTCACTTGT |
| MARKER 108 | AGCCAGATGGAAATACAC | GTGCGATAAAGAGGAGAGT |
| MARKER 109 | TAGTTTTCCCATCACAGAGT | ACAATATTTAGACCTTCCATGA G |
| MARKER 110 | GTGCAGATGCAGATTATATG | CCTTTAGAATTGCCGTATC |
| MARKER 111 | ACAATAACCTGAGACAACAAGA AAC | ATACATCCCCTCCCCTCTCT |

Two bulks were constructed for each of the 12 individuals with each bulk containing 48 markers. The bulked PCR products were then purified using QlAquick PCR Purification columns (QIAGEN) as per manufacturer's instructions. The purified products were then ligated into a pDrive cloning vector (QIAGEN) to allow a universal binding site for the second round PCR. The pDrive vector was selected because of its high efficiency for ligation and due to the fact that it contains the M13 forward and M13 reverse primer-binding sites. The linear vector is designed to exploit the behaviour of *Taq* polymerase, which produces a single adenosine nucleotide overhang on resulting PCR fragments, by containing a complementary base (U-base) at the points of insertion. With a simple ligation reaction the adenosine base from the PCR product and the U-base from the vector ligate together resulting in the recircularisation of the plasmid. This was achieved by adding 5 µl of 2x Ligation Master Mix, 4 µl of PCR product and 1 µl of the pDrive vector (50 ng µL⁻¹) into a 0.2 ml eppendorf tube. Reagents were collected by pulse centrifugation and the ligation reaction was performed at 4°C for approximately 15 h. The ligation product was diluted 1:10 with nanopure water and this formed the template for the second PCR involving a single microsatellite locus specific primer in combination with a fluorescently labelled universal primer M13 (either forward or reverse). The forward M13 (-40) was labelled with the fluorescent dye (FAM) and the reverse with HEX (both supplied by SIGMA ALDRICH). The PCR conditions were the same as in the initial amplification step this time using the diluted ligation product as the DNA template. Products were diluted 1 in 5 and arranged in bulks based on the expected size of fragment and the fluorescent dye used, which allowed numerous samples to be assessed in a single run of the capillary sequencer. These products were separated by capillary electrophoresis on an ABI Prism 3100 sequencer. The sequencer uses a linear flowing media, namely POP-6^{™} polymer (Applied Biosystems), to separate fragments in the capillaries and the fluorescence emitted from the incorporated labelled primer is recorded by the software program Genescan Version 3.1^{™} (Applied Biosystems). The output file allows comparisons of the genetic profiles of individuals by portraying peaks that represent the AFLP-DNA fragments. This fragment analysis was performed using ABI PRISM Genotyper^{®} 3.6NT software (Applied Biosystems), which allows analysis of the size of the fragments (in base pairs) and can also assess the strength of the amplified product. Allele sizes were assessed using ABI PRISM Genotyper^{®} 3.6NT software (Applied Biosystems). Any individual that generated two allelic peaks for any microsatellite marker was deemed partly heterozygous and so discarded as not being a possible haploid or doubled haploid (depending of flow cytometry results).

### Results

### Genome characterisation

A subset of 8 of the 24 candidates identified after the molecular screen including both diploid and haploid individuals (listed in the table below) was subjected to more extensive molecular characterization with 80 additional microsatellite markers using the bulked ligation technique described above (e).

| | **Genotype reference** | **Sample code** | **Ploidy level** |
|---|---|---|---|
| 2 | BATCH 53;03090761C;5 | 060728_0018_01_a | Haploid |
| 4 | BATCH 50;03060260C;2 | 060728_0027_01_a | Haploid |
| 7 | BATCH 53;03080954C;2 | 060728_0035_01_a | Haploid |
| 13 | BATCH 67;0409034MC;4 | 060729_0131_02_a | Haploid |
| 16 | BATCH 65;0409034MC;35 | 060729_0138_02_a | Diploid |
| 18 | BATCH 65;0409034MC;50 | 060729_0141_02_a | Diploid |
| 22 | 0627/125;05090717C;2 | 060731_0085_01_a | Haploid |
| 24 | 0629/97:05100048C;3 | 060731_0105_01_a | Haploid |

As expected, all individuals identified as haploids by flow cytometry contained only a single allele across all 80 loci surveyed. Thus, these individuals are hemizygous for 95 loci in total (including the 15 markers used in the screen) and this was deemed to confirm their haploid status. In contrast, the two diploids were heterozygous for many of the loci screened.

### Verification of the bulked ligation technique

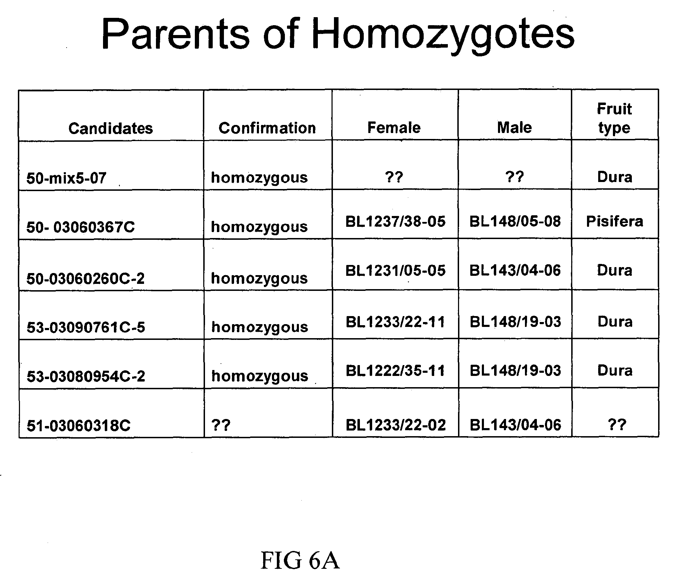

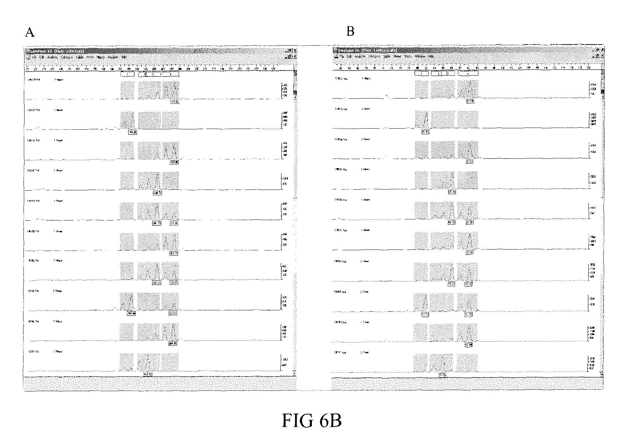

When profiles of ten individuals were subjected to microsatellite analysis by conventional capillary electrophoresis and using labeled microsatellite primers, the profiles obtained indicated identical scores for allelic status across 24 markers (example shown in figure 6B).

Figure 6B shows Electropherograms generated by the bulked-ligation method (A) and conventional microsatellite analysis (B) using the same oil palm genotypes.

### Identification of doubled haploids

Here, we screened all 34 diploid candidates that were homozygous for all 15 markers as described above and applied a further 32 fluorescent labeled microsatellite markers (listed below) using conventional capillary electrophoresis through a ABI Prism 3100 DNA sequencer. There were two genotypes (65-0409034 MC-144 and 65-0409034 MC-114) that were homozygous for all markers. Thus, these plants were homozygous for a total of 47 microsatellite markers and so were deemed to be doubled haploid oil palm plants.

The microsatellites used for this characterization step were as follows:

| **Microsatellite Marker** | **Forward Primer Sequence** | **Reverse Primer Sequence** |
|---|---|---|
| MARKER 16 | GACCTTTGTCAGCATACTTG GTGTG | GCAGGCCTGAAATCCCAAAT |
| MARKER 21 | GGGTAGCAAACCTTGTATTA | ACTTCCATTGTCTCATTATTCT |
| MARKER 23 | TTGCGGCCCATCGTAATC | TCCCTGCAGTGTCCCTCTTT |
| MARKER 29 | GCAAGATGCAATGGAGTTCA | CAAACCGCAGCAAGTCAGA |
| MARKER 36 | CTCCGATGGTCAAGTCAGA | AAATGGGGAAGGCAATAGTG |
| MARKER 44 | ATGACCTAAAAATAAAATCTCAT | ACAGATCATGCTTGCTCACA |
| MARKER 48 | CGGTTTTGTCGCATCTATG | GTCGTCAGGGAACAACAGT |
| MARKER 51 | TCCAAGTAGCAAATGATGAC | TGCCCTGAAACCCTTGA |
| MARKER 54 | GATCCCAATGGTAAAGACT | AAGCCTCAAAAGAAGACC |
| MARKER 55 | TGTGGTTTGAGGCATCTTCT | GCCCACCAAAAGAAAGTAGT |
| MARKER 62 | AGGGAGGCGAACGAGAAACA | CGACTGCTGATGGGGAAGAG |
| MARKER 67 | TTTCTTATGGCAATCACACG | GGAGGGCAGGAACAAAAAGT |
| MARKER 68 | GTTTATCATTTTGGGGTCAG | CGGTGTCCCTCAGGATGTA |
| MARKER 69 | CATGCACGTAAAGAAAGTGT | CCAAATGCACCCTAAGA |
| MARKER 71 | TGTAGGTGGTGGTTAGG | TGTCAGACCCACCATTA |
| MARKER 72 | AGCAAGACACCATGTAGTC | GACACGTGGGATCTAGAC |
| MARKER 74 | GTCCATGTGCATAAGAGAG | CTCTTGGCATTTCAGATAC |
| MARKER 77 | GGGCTTTCATTTTCCACTAT | GCTCAACCTCATCCACAC |
| MARKER 78 | GACAGCTCGTGATGTAGA | GTTCTTGGCCGCTATAT |
| MARKER 79 | ACTTGTAAACCCTCTTCTCA | GTTTCATTACTTGGCTTCTG |
| MARKER 81 | CCACTGCTTCAAATTTACTAG | GCGTCCAAAACATAAATCAC |
| MARKER 84 | GCCTATCCCCTGAACTATCT | TGCACATACCAGCAACAGAG |
| MARKER 88 | TCTCCCAAATCACTAGAC | ATCTGCAAGGCATATTC |
| MARKER 89 | ACGTTTTGGCAACTCTC | ACTCCCCTCTTTGACAT |
| MARKER 92 | AGCAAAATGGCAAAGGAGAG | GGTGTGTGCTATGGAAGATCA TAGT |
| MARKER 96 | TCTATATTTGGTTGGCTTGA | ACTCATTTCAATCTCAGTGTC |
| MARKER 98 | CCTCCACTTCTCTTCATCTT | CTTCCTCAAGCTCAAACAAT |
| MARKER 104 | CCTATTCCTTACCTTTCTGT | GACTTACTATCTTGGCTCAC |
| MARKER 105 | CCTTGCATTCCACTATT | AGTTCTCAAGCCTCACA |
| MARKER 110 | GTGCAGATGCAGATTATATG | CCTTTAGAATTGCCGTATC |
| MARKER 111 | ACAATAACCTGAGACAACAA GAAAC | ATACATCCCCTCCCCTCTCT |
| MARKER 112 | GAACTTGGCGTGTAACT | TGGTAGGTCTATTTGAGAGT |

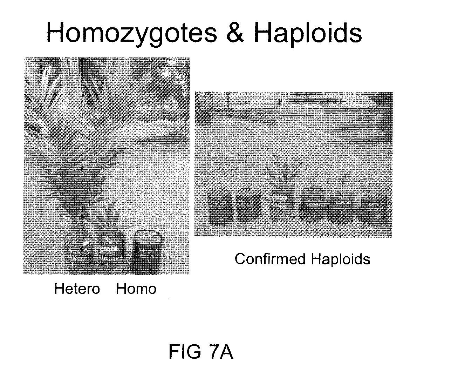

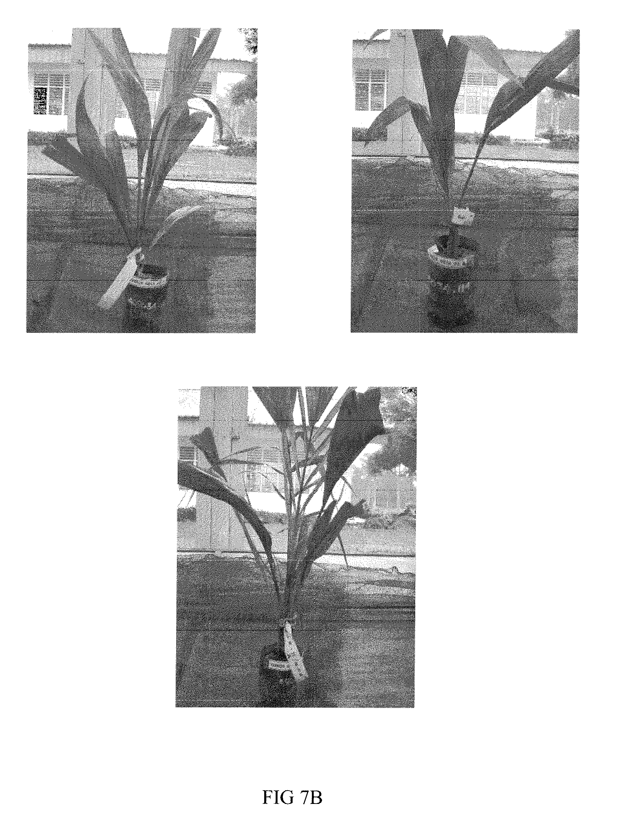

Figure 7B shows images of a typical diploid heterozygous oil palm (bottom) and two doubled haploids (top) sown on the same day.

### Generating doubled haploids from haploids

Haploid cells will sometimes undergo "spontaneous doubling" whereby failure of complete mitosis gives a doubling of the chromosomes. If this occurs early in development, the seed, plantlet and plant derived is a doubled haploid. If no such doubling occurs then a haploid is obtained and in most circumstances, such haploid plants are intrinsically infertile, in that the process of meiosis is unable to generate gametes capable of fertilisation. In order to produce a fertile plant from which sexual progeny can be produced it is necessary either to double the chromosome number of a haploid by application of an external agent, or to rely on the rare process by which a haploid cell can spontaneously double. The former method is the most usually adopted, and usually involves the application of a chemical agent capable of inhibiting mitosis and thereby inducing the formation of a diploid cell. There are several chemicals known to induce such a chromosome doubling process and of these colchicine is the best known, and most commonly utilised. Other similar agents include microtubule inhibitors such as the herbicides trifluralin, and oryzalin. Such chemicals can either be applied to a whole plant and fertile seeds may be produced on that plant, or they can be applied in vitro to isolated cells from which an intact plant can be regenerated using conventional tissue culture techniques. For a full description of available chemical and other methods and their means of application see Kasha (2005) and references therein.

An alternative to the external application of chemicals is the exploitation of spontaneous doubling. For example, in a haploid, the nucleus of an individual cell may occasionally fail to divide normally at mitosis and thus form a diploid cell that ultimately gives rise either to a diploid sector(s) that may encompass most or all of the main shoot axis or (if it occurs in the first embryonic division) a doubled haploid plant. In either case, the selfed seed secured from such individuals will be completely homozygous and genetically identical to the parent. This process can occur during the formation of reproductive cells and in this case it is possible that fertile gametes (pollen or egg cells) may be produced. If both male and female gametes form on the same plant then successful fusion of gametes can take place and an embryo will develop. Such an embryo will be a homozygous diploid, and will breed true in all future selfed generations; all its selfed progeny will be genetically identical. In oil palm, the inflorescences are usually either male or female (though hermaphrodite inflorescences are known to occur occasionally) and therefore selfing of a particular haploid plant may require the storage of pollen from a male inflorescence until a suitable female inflorescence is available for pollination. Such procedures are commonly used in oil palm breeding.

In the present example, it is known that haploid oil palm plants produce their first inflorescences after approximately two years of vegetative growth, and it is likely that such plants will produce a low, but usable frequency of fertile gametes, from which homozygous progeny can be isolated. One haploid plant has now started to flower

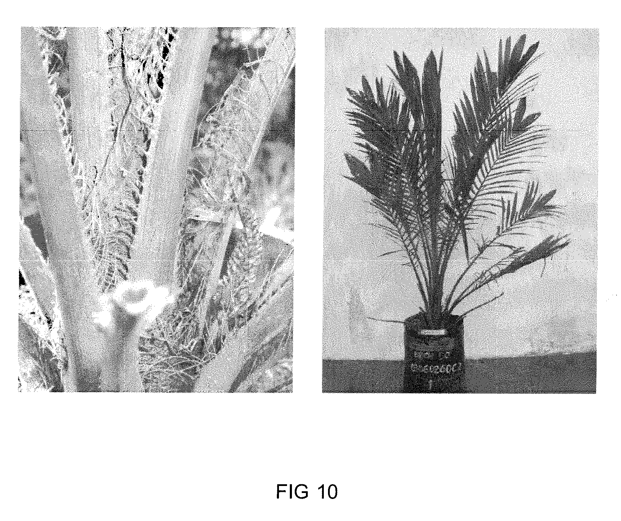

Figure 10 shows confirmed haploid 50-03060260_0002 with first inflorescence two years and seven months after planting (left photograph of inflorescence and right photograph of haploid seedling).

### References

Abdullah R (2005). A decade of oil palm gene manipulation. Where are we now? In: 9th International Conference on Agricultural Biotechnology: Ten Years After. organized by the: International Consortium on Agricultural Biotechnology Research (ICABR) and the: Catholic University of Leuven CEIS-University of Rome "Tor Vergata" Centre of Sustainable Resource Development, University of California at Berkeley Economic Growth Centre, Yale University Ravello (Italy), July 6-10, 2005. pp. 25.
Abdullah R, Zainal A, Heng WY, Li LC, Beng YC, Phing LM, Sirajuddin SA, Ping WYS, Joseph JL, Jusoh SA. (2005). Immature embryo: A useful tool for oil palm (Elaeis guineensis Jacq.) genetic transformation studies. Electronic Journal of Biotechnology ISSN: 0717-3458 Vol.8 No.1, Issue of April 15, 2005. (http://www.ejbiotechnology.info/content/vol8/issue1/full/1/)
Ahloowalia BS, Maluszynski M, Nichterlein K (2004). Global impact of mutation-derived varieties. Euphytica 135: 187-204.
Andersen SB (2005). Haploids in the improvement of woody species. In: Haploids in Crop Improvement II, Vol. 56 (Eds, Palmer C E, Keller WA, Kasha KJ) Springer, Heidelberg, pp. 243-257.
Bains GS, Howard HW. (1950). Haploid plants of Solanum demissum. Nature 166(4227): 795. Barclay, IR (1975). High frequencies of haploid production in wheat (Triticum aestivum) by chromosome elimination. Nature 256: 410-411.
Ben Abdallah A, Lepoivre P, du Jardin P (2001). Apomixis induction possibility explored in date palm (Phoenix dactylifera I.). In: Proceedings Second International Conference on Date Palms (Al-Ain, UAE, March 25-27, 2001) p.164 (http://www.pubhort.org/datepalm/index2.htm)
(b) Bingham ET (1969). Haploids from cultivated alfalfa, Medicago sativa L. Nature 221 (5183): 865-866.
(c) Blakeslee AF, Belling J, Farnham ME, Bergner AD (1922). A haploid mutant in the Jimson weed, Datura stramonium. Science 55: 646-647.
Bohanec B (2003). Ploidy determination using flow cytometry. In: Maluszynski M, Kasha KJ, Forster BP, Szarejko I. (Eds). Doubled Haploid Production in Crop Plants: A Manual. Kluwer Academic Publishers. pp. 397-403.
Bordes J, de Vaulx RD, Lapierre A, Pollacsek M (1997). Haplodiploidization of maize (Zea mays L) through induced gynogenesis assisted by glossy markers and its use in breeding. Agronomie 17 (5): 291-297.
Bordes J, Charmet G, de Vaulx RD, Pollacsek M, Beckert M, Gallais A (2006). Doubled haploid versus S1 family recurrent selection for testcross performance in a maize population. Theor. Appl. Genet. 112(6): 1063-1072.
Bouguedoura, N (1991). Connaissance de la morphogenèse du palmier dattier (Phoenix dactylifer a L.). Etude in situ et in vitro du developpement morphogenetique des appareils végétatif et reproducteur. Thèse de Doctorat d'Etat, Universitee d'Alger, Algeria. (quoted in Loutfi and El Hadrami 2005)
Bouvier L, Zhang YX, Lespinasse Y (1993). Two methods of haploidization in pear, Pyrus communis L.: greenhouse seedling selection and in situ parthenogenesis induced by irradiated pollen. Theor. Appl. Genet. 87(1-2): 229-232.
Brochard P (1981 ). Culture des tissues de palmier dattier. Rapport de recherches 1975-1981. Station Expérimentale de Sidi Mahdi, INRA, Algeria, 96 pp. (quoted in Loutfi and Hadrami 2005)
Chaibi N, Ben Abdallah A, Harzallah H, Lepoivre P (2002) Potentialités androgénétiques du palmier dattier Phoenixdactylifera L. et culture in vitro d'anthères. Biotechnol. Agron. Soc. Environ. 6(4): 201-207.
Chalyk ST (1994). Properties of maternal haploid maize plants and potential application to maize breeding. Euphytica 79(1-2): 13-18.
Chani E, Veilleux RE, Boluarte-Medina T (2000). Improved androgenesis of interspecific potato and efficiency of SSR markers to identify homozygous regenerants. Plant Cell, Tissue and Organ Culture 60: 101-112.
Chase SS (1949). Monoploid frequencies in a commercial double cross hybrid maize, and its component single cross hybrids and inbred lines. Genetics 34: 328-332.
Chaudhari HK (1978). Use of semigamy in the production of cotton haploids. Bulletin of the Torrey Botanical Clu b 105(2): 98-103.
Chaudhari HK (1979). The production and performance of doubled haploids of cotton. Bulletin of the Torrey Botanical Club 106(2): 123-130.
Choo TM (1981). Doubled haploids for studying the inheritance of quantitative characters. Genetics 99: 525-540. Christianson ML, Chiscon MO (1978).Use of haploid plants as bioassay for mutagens. Environ Health Perspect . 27: 77-83.
Clausen RE, Mann MC (1924). Inheritance in Nicotiana tabacum : V. The occurrence of haploid plants in interspecific progenies. Proc. Natl. Acad. Sci. USA 10(4): 121-124.
Coba de la Pena T, Brown S (2001). Flow cytometry. In: Hawes C, Satiat-Jeunemaitre B (eds) Plant Cell Biology 2nd edition. Oxford University Press pp. 85-106. Coconut Research Board (2002). Annual Report of the Coconut Research Board of Sri Lanka. pp. 102. (http://www.treasury.gov.lk/FPPFM/ped/pdfdoc/coconutresearchboard/crb ar2002.pdf)
Coe EH (1959). A line of maize with high haploid frequency. American Naturalist 93: 381-382.
Cooper DC (1943). Haploid-diploid twin embryos in Lilium and Nicotiana. Am. J. Botany. 30: 408-413.
Crow JH (1998). 90 Years ago: the beginning of hybrid maize. Genetics 148: 923-928.
Daker MG (1966). 'Kleine Liebling', a haploid cultivar of Pelargonium . Nature 211 (48): 549-50.
Diemer P, Chinchilla C, Griffee P. Small Holder Oil Palm Manual. (http://ecoport.org/ep?SearchType=earticleView&earticleld=180&page=23 68)
Doctrinal M, Sangwan RS, Sangwan-Norreel BS (1989). In vitro gynogenesis in Beta vulgaris L.: Effects of plant growth regulators, temperature, genotypes and season. Plant Cell, Tissue and Organ Culture 17(1): 1-12.
Dublin P (1972). Polyembryonie et haploïdie chez Theobroma cacao. Café Cacao Thé 16(4): 295-311.
Dublin P, Parvais, J-P (1976). L'haploïde spontanée liée à la polyembryonie chez le Coffea arabica L. Café Cacao Thé 20(2): 83-90.
Dulieu H (1964).[detection of haploid plants among progeny of the cross between Nicotiana tabacum I. and Nicotiana sanderae hort, following irradiation of pollen.][Article in French] C. R. Hebd. SeancesAcad. Sci. 259: 4126-4129.
Duvick DN (2001 ). Biotechnology in the 1930s: the development of hybrid maize. Nature Reviews Genetics 2: 69-73.
Dweikat IM, Lyrene PM (1990). Twin seedlings and haploids in blueberry (Vaccinium spp.). Journal of Heredity 81 (3): 198-200.
Eder J, Chalyk S (2002). In vivo haploid induction in maize. Theor. Appl. Genet . 104: 703-708.
Eeckhaut T, Leus L, Van Huylenbroeck J (2005). Exploitation of flow cytometry for plant breeding. Acta Physiologiae Plantaru m 27 (4B): 743-750.
Eimert K, Reutter G, Strolka B (2003). Fast and reliable detection of doubled-haploids in Asparagus officinalis by stringent RAPD-PCR. Journal of Agricultural Science 141(1): 73-78.
Forster BP, Thomas WTB (2005). Doubled haploids in genetics and plant breeding. Plant Breeding Reviews 25: 57-88.
Gaines EF, Aase HC (1926). A haploid wheat plant. American Journal of Botany 13 (6): 373-385.
Griffis JL, Litz RE (1997). Advances in the in vitro morphogenesis of several coconut: (Cocos nucifera L.) tissues in Florida. In: International Cashew and Coconut Conference, Dar es Salaam. Pp. 349-357. (quoted in Hocher *et al.* 2005)
Grüneberg H. (1936). Haploids in polyembryonic seeds of sea island cotton. J. Hered . 27: 229-232.
Guha S, Maheshwari SC (1964). In vitro production of embryos from anthers of Datura. Nature 204: 497.
Hagberg A, Hagberg G (1980). High frequency of spontaneous haploids in the progeny of an induced mutation in barley. Hereditas 93: 341-343.
Harland SC (1936). Haploids in polyembryonic seeds of Sea Island cotton. Journal of Heredity 27: 229-231.
Hermsen JGT, Ramanna MS (1981 ). Haploidy and plant breeding. Phil. Trans. Royal Soc. Lond. B 292: 499-507.
Hocher V, Verdeil J-L, Malaurie B (2005). Cocos nucifera coconut. In: Biotechnology of Fruit and Nut Crops. Biotechnology in Agriculture Vol. 29. Ed. RE Litz. CABI Publishing, Wallingford ISBN 0 85199 662 0. pp. 90-112.
Hussey G. (1958). An analysis of the factors controlling the germination of the seed of the oil palm, Elais guinee nsis (Jacq.). Annals of Botany 22: 259-284.
Ivanov MA (1938). Experimental production of haploids in Nicotiana rustica L. (and a discussion of haploidy in flowering plants). Genetica 20: 295-397.
Isakov YN, Butorina AK, Muraya LS (1981 ). Discovery of spontaneous haploids in Pinus sylvestris and the prospects of their using in forest genetics and selection. Genetika 17: 701-707.
Johansen DA (1934). Haploids in Hordeum vulgare . Proc. Natl. Acad. Sci. USA 20: 98-100. Jones DF (1917). Dominance of linked factors as a means of accounting for heterosis. Genetics 2: 466-479.
Jones LH (1989). Prospects for biotechnology in oil palm (Elaeis guineensis ) and coconut (Cocos nucifera ) improvement. Biotechnology and Genetic Engineering Reviews 7: 281-296.
Kasha KJ (ed.) (1974). Haploids in Higher Plants. The Office of Continuing Education, University of Guelph, Guelph, Ontario. pp. 421.
Kasha KJ (2005). Chromosome doubling and recovery of doubled haploid plants, In: Haploids in Crop Improvement II, Vol. 56 (Eds, Palmer CE, Keller WA and Kasha KJ) Springer, Heidelberg, pp. 123-152.
Kasha KJ, Kao KN (1970). High frequency haploid production in barley (Hordeum vulgare L.). Nature 225: 874-876.
Kermicle JL (1971 ). Pleiotropic effects on seed development of the indeterminate gametophyte gene in maize. American Journal ofBotany 58(1): 1-7.
Kimber G, Riley R (1963). Haploid angiosperms. Bot Rev. 29: 480-531.
Kostoff D. (1929). An androgenic Nicotiana haploid. Zeitschrift für Zellforschung 9: 391-396.
Kurtar ES, Sari N, Abak K (2002). Obtention of haploid embryos and plants through irradiated pollen technique in squash (Cucurbita pepo L.). Euphytica 127: 335-344.
Kuzuya M, Hosoya K, Yashiro K, Tomita K, Ezura H (2003). Powdery mildew (Sphaerotheca fuliginea ) resistance in melon is selectable at the haploid level. Journal of Experimental Botany 54: 1069-1074.
Lanaud C (1988). Origin of haploids and semigamy in Theobroma cacao L. Euphytica 38 (3): 221 - 228.
Lashermes P, Beckert M. (1988). Genetic control of maternal haploidy in maize (Zea mays L.) and selection of haploid inducing lines. Theoret. Appl. Genet. 76(3): 405 - 410
Lashermes P, Couturon E, Charrier A (1994). Doubled haploids of Coffea canephora : development, fertility and agronomic characteristics. Euphytica 74(1-2): 149-157.
Lee LP, Hecht A (1975). Chloroplasts of monoploid and diploid Oenothera hookeri . American Journal of Botany 62(3): 268-272.
Lesley MM, Frost HB (1928). Two extreme "small" Matthiola plants: a haploid with one and a diploid with two extra chromosome fragments. American Naturalist 62: 22-33.
Lespinasse Y, Godicheau M, Duron M (1983). Potential value and method of producing haploids in the apple tree, Malus pumila (Mill.). Acta Hort. (ISHS) 131: 223-230.
Li M (2005). Anatomische, cytologische und histologische Untersuchungen zur somatischen Variation in verschiedenen Teilklonen von Pelargonium zonale 'Kleiner Liebling'. Dissertation. Berlin, Humboldt-Universität zu Berlin, Landwirtschaftlich-Gärtnerische Fakultät. pp. 107.
Lotfi M, Alan AR, Henning MJ, Jahn MM, Earle ED (2003). Production of haploid and doubled haploid plants of melon (Cucumis melo L) for use in breeding for multiple virus resistance. Plant Cell Rep . 21(11): 1121-1128.
Loutfi K, El Hadrami I (2005). Phoenixdactylifera date palm. In: Biotechnology of Fruit and Nut Crops. Biotechnology in Agriculture Vol. 29. Ed. RE Litz. CABI Publishing, Wallingford ISBN 0 85199 662 0. pp. 144-156.
Luyindula N, Mantantu N, Dumortier F, Corley RHV (2005). Effects of inbreeding on growth and yield of oil palm - Inbreeding of oil palm. Euphytica 143(1-2): 9-17.
Madon M, Clyde MM, Hashim H, Yusuf MY, Mat H, Saratha S (2005a). Polyploidy induction of oil palm through colchicine and oryzalin treatments. Journal of Oil Palm Research 17: 110-123.
Madon M, Heslop-Harrison JS, Schwarzacher T, Mohd Rafdi MH, Clyde MM (2005b). Short communication: cytological analysis of oil palm pollen mother cells (PMCs). Journal of Oil Palm Research 17: 176-180.
Madon M, Clyde MM, Rafdhi MM, Heslop-Harrison P, Schwarzacher T (2006). Initial efforts on the production of oil palm (Elais guineensis) haploids. p. 41. In: The International Conference "Haploids in Higher Plants III" Programme and Abstracts. Vienna, Austria. Feb 12-15, 2006. Abstract N7. p. 41.
Maluszynski M, Kasha KJ, Forster BP, Szarejko I (Eds). (2003a). Doubled Haploid Production in Crop Plants: A Manual. Kluwer Academic Publishers. pp. 480.
Maluszynski M, Kasha KJ, Szarejko I (2003b). Published double haploid protocols in plant species. In: Haploid Production in Crop Plants: A Manual. Maluszynski M, Kasha KJ, Forster BP, Szarejko I (Eds). Kluwer Academic Publishers. pp. 309-335.
Marks GE (1973). Selecting asparagus plants as sources of haploids. Euphytica 22(2): 310-316.
Martínez-Gómez P, Arulsekar S, and Gradziel TM (2002). Characterization of twin embryos in almond. Acta Hort. (ISHS) 591: 257-262.
McCray FA (1932). Another haploid Nicotiana tabacum plant. Bot. Gaz. 93: 227-230.
Medrano H, Primo-Millo E (1985). Selection of Nicotiana tabacum haploids of high photosynthetic efficiency. Plant Physiol . 79(2): 505-508.
Melchers G (1972). Haploid higher plants for plant breeding. Zeitschrift für Pflanzenzüchtung 67(1): 19-32.
Metwally El, Moustafa SA, El-Sawy BI, Haroun SA, Shalaby TA (1998). Production of haploid plants from in vitro culture of unpollinated ovules of Cucurbita pepo . Plant Cell, Tissue and Organ Culture 52(3): 117 - 121.
Monfort S (1984). Recherche d'une méthode d'obtention d'haploïdes in vitro de Cocos nucifera L. in vitro. Thèse de doctorat en sciences, Université de Paris-Sud Centre d'Orsay (France).
Monfort S (1985). Androgenesis of coconuts: embryos from anther culture. ZeitschriftfürPflanzenzüchtung 94: 251-254.
Morgan DT (1976). Monoploids in Zea mays L. following crosses with untreated and X-rayed pollen. Genetica 46(2) 133-138.
Morgan DT, Rappleye RD (1954). A cytogenetic study on the origin of multiple seedlings of Capsicum frutescens. American Journal of Botany 41 (7): 576-585.
Muren RC (1989). Haploid plant induction from unpollinated ovaries in onion. Hortscience 24(5): 833-834.
Naess SK, Swartz HJ, Bauchan GR (1998). Ploidy reduction in blackberry. Euphytica 99(1 ): 57-73.
Nei M (1963). The efficiency of haploid methods of plant breeding. Heredity 18: 95-100.
Ninan CA, Raveendranath (1965). A naturally occurring haploid embryo in coconut palm (Cocos nucifera L.). Caryologia 18(4): 619-623.
Palmer, CE, Keller, WA (2005a). Overview of haploidy, In: Haploids in Crop Improvement II, Vol. 56 (Eds, Palmer, CE, Keller, WA and Kasha, KJ) Springer, Heidelberg, pp. 3-9.
Palmer CE, Keller WA (2005b). Challenges and limitations to the use of haploidy in crop improvement. In: Haploids in Crop Improvement II , Vol. 56 (Eds, Palmer CE, Keller WA and Kasha KJ) Springer, Heidelberg, pp. 295-303. Pannetier C, Buffard-Morel J (1986). Coconut Palm (Cocos nucifera L.). In: Biotechnology in Agriculture and Forestry. Vol. 1. Trees 1. (Ed. YPS Bajaj). Springer-Verlag, Berlin. pp.430-450.
Perera PIP (2002a). Studies on the pollen development of Cocosnucifera L. cv Sri Lanka Tall (Coconut variety 'Sri Lanka Tall') for haploid culture. In: Proceeding of Sri Lanka Association for the Advancement of Science SLAAS. Colombo 2002. Abstract 131 B. p. 45.
Perera PIP (2002b). Cytological examination of pollen development for anther culture of coconut (Cocos nucifera L.) Sri Lanka Tall. Cocos 14: 45.
Perera PIP (2003). Cytological examination of microspore development for microspore and anther culture of coconut (Cocos nucifera L.) cv Sri Lanka Tall. Cocos 15: 53-59.
Perera PIP, Hocher V, Verdeil J-L, Weerakoon, LK, Yakandawala DMD (2006). Recent advances in anther culture of coconut (Cocos nucifera L.). Abstract S-120. In: Biotechnology and Sustainable Agriculture 2006 and Beyond. 11th International Association for Plant Tissue Culture & Biotechnology. Beijing, China. Abstract Book p. 45.
Pohlheim F (1968). Thuya gigantea gracilis Beissn. - a haploid gymnosperm. Biol. Rundschau 6: 84-86.
Prakken R (1943). A spontaneous haploid of Nicotiana Tabacum . Genetica 23(1 ): 63 - 76. Randall TE, Rick CM (1945). A cytogenetic study of polyembryony in Asparagus officinalis L. American Journal of Botany 32(9): 560-569.
Rees AR. (1962). High-temperature pre-treatment and the germination of seed of the oil palm, Elaeis guineensis (Jacq.). Annals of Botany 26: 569-581.
Rival A, Parveez GKA (2005). Elaisguineensis oil palm. In: Biotechnology of Fruit and Nut Crops. Biotechnology in Agriculture Vol. 29. Ed. RE Litz. CABI Publishing, Wallingford ISBN 0 85199 662 0. pp. 113-143.
Rongbai L, Pandey MP, Pandey SK, Dwivedi DK (1999). Agro-morphological characterization of ovary culture-derived plants of rice (Oryza sativa L.) Euphytica 106(3): 197-203.
Shull GH (1908). The composition of a field of maize. Am. Breeders Assoc. Rep. 4: 296-301.
Sidhu PK, Howes NK, Aung T, Zwer PK, Davies PA (2006). Factors affecting oat haploid production following oat x maize hybridization. Plant Breeding 125: 243-247.
Snape JW (1989). Doubled haploid breeding: theoretical basis and practical applications, I n: Second International Symposium on Genetic Manipulation in Crops (Eds, Mujeeb-Kazi A, Sitch LA) International Maize and Wheat Improvement Center, El Batan, pp. 19-30.
Sounigo O, Lachenaud P, Bastide P, Cilas C, N'Goran J, Lanaud C (2003). Assessment of the value of doubled haploids as progenitors in cocoa (Theobroma cacao L.) breeding. J. Appl. Genet. 44(3): 339-353.
Sreenivasan MS, Mamachandran M, Sundar KR (1982). Frequency of polyploids in Coffea arabica L.. In: Vishveshwara S (ed) Proc 4th Annual Symposium on Plantation Crops, Mysore, India. Placrosym 4: 23-28. Srisawat T, Kanchanapoom K (2005). The influence of physical conditions on embryo and protoplast culture in oil palm (Elaeis guineensis Jacq.). ScienceAsia 31: 23-28.
Srisawat, T., Kanchanapoom, K., Pattanapanyasat, K., Srikul, S, Chuthammathat, W. (2005). Flow cytometric analysis of oil palm: a preliminary analysis for cultivars and genomic DNA alteration. Songklanakarin J. Sci. Technol . 27(Suppl. 3): 645-652.
Stettler RF, Howe GE (1966). The production of homozygous tree material In: Joint Proceedings of the Second Genetics Workshop of the Society of American Foresters and the Seventh Lake States Forest Tree Improvement Conference; Res. Pap. NC-6. St. Paul, MN: U.S. Department of Agriculture, Forest Service, North Central Forest Experiment Station. 67-69.
Suenaga K (1994). Doubled haploid system using the intergeneric crosses between wheat (Triticum aestivum) and maize (Zea mays). Bull. Natl. Inst. Agrobiol. Resour. 9: 83-139.
Tang F, Tao Y, Zhao T, Wang G (2006). In vitro production of haploid and doubled haploid plants from pollinated ovaries of maize (Zea mays ). Plant Cell, Tissue and Organ Culture 84(2): 100210-100214.
Te-chato S, Hilae A, Moosikapala L (2005). Microcolony formation from embryogenic callus-derived protoplasts of oil palm. Songklanakarin J. Sci. Technol. 27(4): 685-691.
Texeira JB, Sondahl MR, Kirby EG (1994). Somatic embryogenesis from immature inflorescences of oil palm. Plant Cell Reports 13: 247-250.
Thanh-Tuyen NT (1985). Anther culture: its prospects for coconut improvement. Philip pine Journal of Crop Science 10: 764-771.
Thanh-Tuyen NT (1990). Coconut (Cocos nucifera L.): anther culture. In: Biotechnology in Agriculture and Forestry. Vol. 10. Legumes and Oilseed Crops I. Bajaj YPS (ed.) Springer-Verlag, Berlin. pp. 555-568.
Thanh-Tuyen NT, De Guzman E. (1983a). Pollen development stages for coconut anther culture. Kalikasan, Philippine Journal of Biology 12: 135-144.
Thanh-Tuyen NT, De Guzman E. (1983b). Formation of pollen embryos in cultured anthers of coconut (Cocos nucifera L.). Plant Science Letters 29(1): 81-88.
Thomas WTB, Foster BP, Gertsson B (2003). Doubled haploids in plant breeding. In: Haploid Production in Crop Plants: A Manual. Maluszynski M, Kasha KJ, Forster BP, Szarejko I (Eds). Kluwer Academic Publishers. pp. 337-349.
Todorova M, Ivanov P, Shindrova P, Christov M, Ivanova I. (1997). Doubled haploid production of sunflower (Helianthusannuus L.) through irradiated pollen-induced parthenogenesis. Euphytica 97(3): 249-254.
Tosca A, Arcara L, Frangi P (1999). Effect of genotype and season on gynogenesis efficiency in Gerbera. Plant Cell, Tissue and Organ Culture 59(1): 77-80.Uijtewaal BA, Huigen DJ, Hermsen JGT (1987). Production of potato monohaploids (2n=x=12) through prickle pollination. Theoret. Appl. Genet. 73(5): 751-758.
Uno Y, li Y, Kanechi M, Inagaki N (2002). Haploid production from polyembryonic seeds of Asparagus officinalis L. Acta Hort. (ISHS) 589: 217-224.
Van Geyt J, Speckmann GJ, Halluin KD, Jacobs M (1987). In vitro induction of haploid plants from unpollinated ovules and ovaries of the sugarbeet (Beta vulgaris L.). Theoret. Appl. Genet . 73: 920-925.
Vaucheret H, Mourrain P, Robalo JC, Pollien JM (1995). Nitrite reductase silencing as a tool for selecting spontaneous haploid plants. Plant Cell Reports 15(1-2): 12-16.
Wahid MB, Abdullah SNA, Henson, IE (2004). Oil Palm - Achievements and Potential. In: "New directions for a diverse planet". Proceedings of the 4th International Crop Science Congress, 26 Sep - 1 Oct 2004, Brisbane, Australia. Web site www.cropscience.org.au
Whitehead RA, Chapman GP (1962). Twinning and haploidy in Cocos nucifera. Nature 195: 1228-1229.
Yang HY, Zhou C. (1982). In vitro induction of haploid plants from unpollinated ovaries and ovules. Theoret. Appl. Genet. 63(2): 97-104.
Zhang YX, Lespinasse Y (1991). Pollination with gamma-irradiated pollen and development of fruits, seeds and parthenogenetic plants in apple. Euphytica 54(1): 101-109.

## Claims

1. A method for selecting haploid individuals or homozygous doubled haploid plants for perennial crops, including:- palms, rubber, mango, emping, soursop, coffee, cocoa, tea, durian, top fruit (apples, pears, cherries), stone fruit and berries the method comprising:
(a) providing a population of seeds, seedlings or plants;
(b) choosing individuals with an atypical phenotype;
(c) assessing the heterozygosity of the chosen seed or germinating seedling in a prescreen that uses one or more molecular, biochemical or phenotypic markers
(d) assessing the DNA content of a somatic cell of a chosen individuals not identified as heterozygous in step (c); and optionally
(e) further assessing the homozygosity of the chosen seed or germinating seedling using multiple molecular markers;
(f) selecting a chosen individuals identified as highly homozygous on the basis of step (c) or (e) and/or as haploid on the basis of step (d); or highly homozygous on the basis of step (c) or (e) and diploid on the basis of step (d)

2. The method of claim 1 for selecting a haploid plant wherein step (f) is selecting a chosen individual that is identified as putatively completely hemizygous on the basis of lacking multiple alleles for all markers assessed in steps (c and e) and haploid on the basis of step (d).

3. The method of claim 1 for selecting a homozygous doubled haploid plant wherein step (f) is selecting a chosen germinated seedling that is identified as lacking any signs of heterozygosity on the basis of exhibiting single alleles for all markers assessed in steps (c and e) and diploid on the basis of step (d).

4. The method according to any one of claims 1 to 3, wherein the heterozygosity prescreen step of using molecular, biochemical or phenotypic markers to discard heterozygous individuals using multiple co-dominant molecular markers, for example between 2 to 100 microsatellite markers or Sequenced Characterised Polymorphic Regions (SCAR) markers or Single Nucleotide Polymorphism (SNP) markers.

5. The method according to any one of claims 1 to 4, wherein the atypical phenotype is an atypical growth morphology.

6. The method according to claim 5 wherein the atypical growth morphology of a seed or seedling is one or more of reduced radicle growth, altered radicle:plumule length ratio, changed radicle:plumule angle, altered colour of radicle or plumule, altered seed shape or size and altered radicle width:length ratio, or another aspect of physical phenotype.

7. The method according to any one of claims 1 to 4 wherein the atypical phenotype is germination of two embryos from a single seed.

8. The method according to any one of the preceding claims wherein the step of further assessing the homozygosity of the chosen germinated seedling using multiple molecular markers comprises using between 50 and 200, for example between 70 and 120 microsatellite markers.

9. The method according to any one of the preceding claims wherein the step of further assessing the homozygosity of the chosen germinated seedling using multiple molecular markers is performed on a pooled sample from multiple chosen germinated seedlings.

10. The method of any one of the preceding claims wherein a chosen seed or seedling or plant is identified as lacking apparent heterozygosity if the individual yields only one allele per locus for each molecular marker used.

11. The method according to any one of the preceding claims wherein the population of germinated seedlings comprises at least 1,000,000 seeds, seedlings or plants.

12. The method of claim 11 wherein the population of germinated seeds, seedlings or plants comprises between 5,000,000 and 20,000,000 individuals.

13. The method according to any one of the preceding claims, wherein the population of germinated seedlings is a population of plants.

14. The method according to any of the preceding claims, wherein the perennial crop is a member of the palm family.

15. The method according to Claim 14, wherein the perennial crop is oil palm.

16. A plant selected by the method according to any one of claims 1 to 15.

17. A method for producing a homozygous doubled haploid plant, the method comprising:
(a) selecting a haploid plant using a method according to any one of claims 1, 2, 4 to 15;
(b) obtaining a doubled haploid plant through spontaneous chromosome doubling; or by doubling the chromosome number by application of an external agent to the haploid plant; or by application of an external agent to a cell or cells isolated from the haploid plant, followed by regeneration of a plant using tissue culture; or by selfing the haploid plant.

18. A method for producing a diploid plant, the method comprising:
(a) selecting at least one homozygous doubled haploid plant using a method according to any one of claims 1, 3 to 15; or obtaining at least one homozygous doubled haploid plant using a method according to claim 17;
(b) selfing at least one homozygous doubled haploid plant or intercrossing two homozygous doubled haploid plants.

19. A method specifically for identifying doubled haploid plants comprising:
(a) Identify 20 heterozygous unlinked loci in the maternal parent, preferably using co-dominant molecular markers such as microsatellites or SNP-based markers
(b) Preferably perform a preliminary screen using 1-5 of the selected markers and discard heterozygotes; retain the remainder as candidate doubled haploids
(c) Apply flow cytometry to the retained candidates and discard haploids; retain diploids as potential doubled haploids
(d) Apply the remaining markers to the retained candidates. Individuals that are diploid and homozygous for all 20 loci are deemed doubled haploids (the probability of this occurring by independent assortment is 2²⁰=1/1,048,576)

20. A plant produced by the method according to any one of claims 17 to 19.

21. A haploid oil palm plant.

22. A homozygous doubled haploid oil palm plant.

23. The homozygous doubled haploid oil palm plant of claim 22 wherein said plant is homozygous for at least 50, 70, 80, 90 or 100 microsatellite markers.

24. An F₁ hybrid oil palm plant

25. The fruits and the extracted products, including oil, from an F₁ oil palm plant.

26. A method of obtaining palm oil by extraction from an F1 hybrid created from homozygous doubled haploid parents.

27. A method for selecting haploid individuals or homozygous doubled haploid plants for perennial crops, including:- palms, rubber, mango, emping, soursop, coffee, cocoa, tea, durian, top fruit (apples, pears, cherries), stone fruit and berries the method comprising:
(a) providing a population of seeds, seedlings or plants;
(b) choosing individuals with an atypical phenotype;
(c) assessing the heterozygosity of the chosen seed or germinating seedling in a prescreen that uses one or more molecular, biochemical or phenotypic markers;
(d) assessing the DNA content of a somatic cell of a chosen individuals not identified as heterozygous in step (c); and
(e) selecting a chosen individuals identified as highly homozygous on the basis of step (c) and/or as haploid on the basis of step (d); or highly homozygous on the basis of step (c) and diploid on the basis of step (d)

28. A method according to Claim 27 further comprising the step of assessing the homozygosity of the chosen seed or germinating seedling using multiple molecular markers in order to identify highly homozygous individuals, said step to be carried out between step (d) and step (e)
